(19) Europäisches Patentamt / European Patent Office / Office européen des brevets

(11) **EP 2 609 856 A2**

(12) **EUROPEAN PATENT APPLICATION**

(43) Date of publication:
**03.07.2013 Bulletin 2013/27**

(51) Int Cl.:
**A61B 5/053** (2006.01)          **A61B 5/00** (2006.01)

(21) Application number: **12199439.6**

(22) Date of filing: **27.12.2012**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(30) Priority: **28.12.2011 JP 2011288528**<br>       **19.12.2012 JP 2012276570** | (71) Applicant: **Tanita Corporation**<br>**Tokyo 174-8630 (JP)**<br><br>(72) Inventor: **OZAWA, Satoko**<br>**Itabashi-ku, Tokyo 174-8630 (JP)**<br><br>(74) Representative: **Grünecker, Kinkeldey,**<br>**Stockmair & Schwanhäusser**<br>**Leopoldstrasse 4**<br>**80802 München (DE)** |

(54) **Body condition information processing apparatus, non-transitory computer readable recording medium, and method for processing body condition information**

(57)    A condition information processing apparatus (100A) includes: an obtaining section (31) that obtains information relating to a bioelectrical impedance measured by means of impressing an alternating current of a predetermined frequency to a body surface of a user. An operating section (38) calculates an index relating to a body condition on the basis of the information relating to the bioelectrical impedance.

FIG. 1

EP 2 609 856 A2

**Description**

BACKGROUND OF THE INVENTION

1. Field of the Invention

The present invention relates to techniques for processing information relating to the condition of a user's body on the basis of information relating to bioelectrical impedance of the user's body.

2. Description of Related Art

There are known cases in which a person who plays sports, such as an athlete, cannot demonstrate the person's true ability due to injury, poor condition, or overtraining, Thus, it is important to assess the condition of the person's body in advance to get into shape. With regard to the assessment of the condition of the athlete, the athlete depends mostly on subjective assessments such as the athlete's own assessment, a trainer's assessment and the like. There is a case in which an athlete cannot help but hiding a poor condition only because the athlete wishes to take part in a competition. Since a trainer takes care of a number of athletes, it is difficult for the trainer to manage the conditions of all of them.
As for technology for assessing physical condition objectively, for example, there is a blood testing method in which taking a blood sample, which is invasive, is performed (e.g., patent document 1).
In this blood testing method, the number of red blood cells, white blood cells, and blood platelets, which are blood corpuscle components in blood obtained by means of the blood sampling, and the concentration of hemoglobin included in the red blood cells, etc. are measured, so that the condition of a user is assessed.
Patent Document 1: Japanese Laid Open Patent Application No. 2005-77148

SUMMARY OF THE INVENTION

However, since an apparatus for sampling blood weighs dozens of kilograms and needs reagents such as dilute solutions, hemolysis reagents, etc., it is installed in a place such as a laboratory, etc. Thus, in an invasive method such as blood sampling, a subject must go to the laboratory directly, or it is necessary to bring a blood sample in a hospital room to the laboratory in some way. Accordingly, it is difficult to continuously monitor physical conditions on a daily basis.
In the measurement of body fat percentage, muscle mass, amount of fluid inside a cell, and amount of fluid outside a cell by using a bioelectrical impedance method, which is a non-invasive method, it has not yet been possible to measure conditions in a body on the basis of muscle fatigue, injury, disease, etc.
In order to solve the above-described problems, the present invention provides a condition information processing apparatus, a non-transitory computer readable recording medium for a condition information processing apparatus, and a method for processing condition information, in which it is possible to assess conditions in a non-invasive method easily and objectively.
A condition information processing apparatus (100) includes: an obtaining section (31) that obtains information relating to a bioelectrical impedance measured by means of impressing an alternating current of a predetermined frequency at a body surface of a user; and an operating section (38) that calculates an index relating to a body condition on the basis of the information relating to the bioelectrical impedance.
In the present invention, the information relating to the bioelectrical impedance includes a voltage, a bioelectrical impedance, a ratio of bioelectrical impedances, and a ratio of a reactance component and a resistance component in the bioelectrical impedance. The index relating to the condition includes, for example, a deviation value of a ratio of extracellular fluid and intracellular fluid. The difference between a measured value of the ratio and an average value of the ratio is divided by a standard deviation to normalize the ratio. Then, the normalized ratio is multiplied by 10. The deviation value is obtained by adding 50 to the result of the multiplication. The deviation value is used as an index relating to the condition, so that it is possible to determine the quantity of the fatigue of muscle, inflammation, and swelling.
In the present invention, the ratio of extracellular fluid and intracellular fluid in a normal condition, and the ratio of extracellular fluid and intracellular fluid upon injury or disease, etc., are obtained. It is possible to calculate the index relating to the body condition on the basis of the obtained ratios. Therefore, if the index relating to the condition is used, it is possible to estimate the condition easily and objectively.
In an aspect of the present invention, the condition information processing apparatus (100) further includes a condition estimating section (38) that estimates the body condition on the basis of a newly calculated index relating to the body condition and a past index relating to the body condition recorded as a measurement history.
According to this aspect, it is possible to analyze the tendency of the change of the user's condition on the basis of the newly calculated index relating to the condition and the past index relating to the condition. For example, even if the newly calculated index is lower than a predetermined value, or the newly calculated index is higher than a predetermined value, in the case in which the newly calculated index continuously declines compared to the previous value two or more times, there might be change in the user's body of which the user is unaware. However, according to the present invention, it is possible to estimate that the user is in poor condition to prevent injury or disease.

In an aspect of the present invention, the condition information processing apparatus (100) further includes: a pair of current applying electrodes (23a, 23b) that is capable of contacting the body surface; a pair of voltage measuring electrodes (24a, 24b) that is capable of contacting the body surface; an alternating current applying section (32b) that impresses an alternating current to the current applying electrodes; a frequency setting section (32c) that sets a predetermined frequency as a frequency of the alternating current impressed by the alternating current applying section; and a voltage measuring section (33) that measures a voltage at the time of impressing the alternating current of the predetermined frequency, in which the obtaining section (31) obtains either of the voltage measured by the voltage measuring section and the bioelectrical impedance as the information relating to the bioelectrical impedance.

According to this aspect, a voltage, at the time of impressing the alternating current of a predetermined frequency by a pair of current applying electrodes, is measured by a pair of voltage measuring electrodes. The bioelectrical impedance is calculated on the basis of the measured voltage. The ratio of extracellular fluid and intracellular fluid in normal condition, or the ratio of extracellular fluid and intracellular fluid in injury or disease is obtained on the basis of the bioelectrical impedance. It is possible to calculate the index relating to the body condition on the basis of the obtained index. Therefore, if the index relating to the condition is used, it is possible to estimate the condition easily and objectively.

In an aspect of the present invention, the condition information processing apparatus (100) further includes: the obtaining section (31) obtains either of: a) the voltage measured by means of impressing the alternating current of a predetermined low frequency and the alternating current of a predetermined high frequency, and b) the bioelectrical impedance as the information relating to the bioelectrical impedance, and the operating section (38) calculates the index relating to the body condition on the basis of a ratio of the bioelectrical impedance at the time of impressing the alternating current of high frequency and the bioelectrical impedance at the time of impressing the alternating current of low frequency.

According to this aspect, the ratio of the bioelectrical impedance at the time of impressing the alternating current of low frequency to the bioelectrical impedance at the time of impressing the alternating current of high frequency is calculated. If the alternating current is used, the resistance of the extracellular fluid, the resistance of the intercellular fluid, and the electrical capacity of cell membrane are current paths, so that the alternating current of low frequency does not flow into a condenser of the cell membrane formed by lipid bilayer, but most of the impressed alternating current flows into the extracellular fluid. Since the alternating current of high frequency is not affected by the cell membrane, the alternating current flows into the extracellular fluid and the intercellular fluid. Therefore, if the alternating current of low frequency is used, information of only the extracellular fluid is reflected. If the alternating current of high frequency is used, information of the resistance of the intercellular fluid is reflected more than the other elements. If injury or disease occurs, or the fatigue of muscles is accumulated, inflammation or swelling occurs in the body. If the inflammation or swelling occurs, intercellular fluid flows out of the cell membrane. Furthermore, the amount of only the extracellular fluid is increased whether or not the amount of the intercellular fluid changes. As a result, a ratio of the extracellular fluid to the entirety of the cell is increased. Therefore, according to this aspect, the ratio of the bioelectrical impedance at the time of impressing the alternating current of high frequency and the bioelectrical impedance at the time of impressing the alternating current of low frequency is calculated, so that the ratio of extracellular fluid and intracellular fluid in a normal condition, or the ratio of extracellular fluid and intracellular fluid in injury or disease is obtained. It is possible to calculate the index relating to the body condition on the basis of the obtained ratio. It is possible to estimate the condition easily and objectively.

In an aspect of the present invention, the obtaining section (31) obtains either of a) the voltage measured by means of impressing an alternating current of a predetermined single frequency, and b) a ratio of a resistance component and a reactance component in a bioelectrical impedance on the basis of the voltage, and the operating section (38) that calculates the index relating to the body condition on the basis of the ratio of the resistance component and the reactance component in the bioelectrical impedance.

According to the present invention, the ratio of the resistance component and the reactance component in the bioelectrical impedance at the time of impressing the alternating current of a predetermined frequency is calculated. If the alternating current is used, the resistance of the extracellular fluid, the resistance of the intercellular fluid, and the electrical capacity of cell membrane are current paths. The resistance of the extracellular fluid and the resistance of the intercellular fluid become the resistance component. The electrical capacity of cell membrane becomes the reactance component. Since the predetermined frequency is used as that of the alternating current, and the ratio of the resistance component and the reactance component is calculated, it is possible to obtain information of the extracellular fluid, the intercellular fluid, and cell membrane.

If injury or disease occurs, or fatigue in the muscles builds up, inflammation or swelling occurs in the body. If the inflammation or swelling occurs, intercellular fluid flows out of the cell membrane. Furthermore, the amount of only the extracellular fluid is increased whether or not the amount of the intercellular fluid changes. As a result, a ratio of the extracellular fluid to the entirety of the cell is increased.

In the present invention, the ratio of the resistance component and the reactance component by using the alternating current of the predetermined frequency is calculated, so that the ratio of extracellular fluid and intracellular in a normal condition, or the ratio of extracellular fluid and intracellular upon injury or disease is obtained. It is possible to calculate the index relating to the body condition on the basis of the obtained ratio. It is possible to estimate the condition easily and objectively.

In an aspect of the present invention, the operating section (38) refers to additional information in the measurement history, in which the calculated indexes relating to the condition and the addition information associated with the index, and aggregates the indexes in the measurement history, which satisfy a predetermined condition, to calculate the index.

According to this aspect, the index is calculated by referring to the addition al information, and aggregating the indexes in the measurement history, which satisfy a predetermined condition. The additional information includes, for example, the subjective information input by the operation of the user, and the objective information such as information on whether or not injury or disease occurred. Therefore, it is possible to refer to the relationship between the index relating to the body condition, and the subjective information and the objective information as the additional information. It is possible to estimate the condition more precisely.

In an aspect of the present invention, the additional information includes: a) an objective information including at least one of the user's weight, body temperature, and blood pressure at the time of calculating the index relating to the condition; and b) a subjective information input on the basis of an operation by the user, and the operating section (38) appropriately selects the objective information and the subjective information, so that the operating section aggregates the indexes associated with at least one of the selected objective information and the selected subjective information in the measurement history.

According to this aspect, if the additional information is the subjective information input by the operation of the user, for example, it is possible to compare the subjective information to the objective information. If the additional information is objective information such as the user's weight, information on whether or not injury or disease occurred, it is possible to take into account the relevance to the measured index, so that the estimation of the body condition is executed more easily.

The objective information includes specific numeral data, which is measured by another measuring device. It may be directly input from another measuring device. The user may input the numeral data manually. If the data is numeric data, which can be measured by a measuring device, the results of sports, such as times, may be included in the objective information. The subjective information is information on the basis of the feeling of the user or people around the user such as a body condition, feeling, complexion, and information on whether or not injury or disease occurs. It is possible to input text data of "Cheerful" or "Poor Condition" as the subjective information. The subjective information may include a gradual evaluation data such as Good, Normal, or Poor, which can be freely selected by the user.

In this case, it is possible to filter or sort the measured data of the bioelectrical impedance on the basis of the additional information. It is possible to narrow and extract freely chosen data. As a result, for example, it is possible to study correlation between the objective information and the subjective information, which is input on the basis of the operation of the user. It is possible to calculate the average or the rate of deviation of the ratio of extracellular fluid and intracellular fluid, which are extracted on the basis of the condition. Therefore, it is possible to estimate the body condition easily.

A non-transitory computer readable recording medium in which a program causing a computer to function as: an obtaining section (31) that obtains information relating to a bioelectrical impedance measured by means of impressing an alternating current of a predetermined frequency to a body surface of a user; and an operating section (38) that calculates an index relating to a body condition on the basis of the information relating to the bioelectrical impedance.

In an aspect of the present invention, the program further causes a computer to function as: a condition estimating section (38) that estimates the body condition on the basis of a newly calculated index relating to the body condition and a past index relating to the body condition recorded as a measurement history.

The program may be stored in a recording medium. If the recording medium is used, it is possible to install the program in the computer. The recording medium, in which the program is recorded, may be a non-transitory recording medium such as a CD-ROM.

A method for processing condition information includes: obtaining information relating to a bioelectrical impedance measured by means of impressing an alternating current of a predetermined frequency to a body surface of a user; and calculating an index relating to a body condition on the basis of the information relating to the bioelectrical impedance.

In an aspect of the present invention, the method further includes estimating the body condition on the basis of a newly calculated index relating to the body condition and a past index relating to the body condition recorded as a measurement history.

In an aspect of the present invention, the method further includes contacting a pair of current applying electrodes to the body surface; contacting a pair of voltage measuring electrodes to the body surface; applying an alternating current to the current applying electrodes; setting a predetermined frequency as a frequency of the impressed alternating current; and measuring a voltage at the time of impressing the alternating current of the predetermined frequency; obtaining either of the voltage measured by the voltage measuring section and the bioelectrical impedance as the information relating to the bioelectrical impedance.

According to this aspect, the bioelectrical impedance of the body is measured, when assessing the condition of the body, and it is possible to calculate the index relating to the condition in non-invasive method easily and objectively.

BRIEF DESCRIPTION OF THE DRAWINGS

Fig. 1 is an outside view of a condition information processing apparatus according to a first embodiment of the present invention;

Fig. 2 is a block diagram showing detailed structure of the condition information processing apparatus shown in Fig. 1;

Fig. 3 is a model diagram modeling a current path of a living body with an electrical equivalent circuit;

Fig. 4 is a model diagram of a cell showing a current path for each frequency in an electrolyte tissue mainly indicating a muscle tissue;

Fig. 5 is a model diagram for explaining the relationship between a volume and a bioelectrical impedance;

Fig. 6 is a section diagram of a model of muscle for explaining the increase of an amount of extracellular fluid in the entirety of a muscle tissue due to injury or disease;

Fig. 7 is a flow chart showing a method for assessing condition in the first embodiment;

Fig. 8 is a graph chart showing a condition score of a user in chronological order;

Fig. 9 is a graph chart showing a condition score of a user in chronological order;

Fig. 10 is a graph chart showing a condition score of a user in chronological order;

Fig. 11 is a graph chart showing a condition score of a user in chronological order;

Fig. 12 is a graph chart showing a condition score of a user in chronological order;

Fig. 13 is a graph chart showing a condition score of a user in chronological order;

Fig. 14 is a graph chart showing a condition score of a user in chronological order;

Fig. 15 is a graph chart showing a condition score of a user in chronological order;

Fig. 16 is a graph chart showing a condition score of a user in chronological order;

Fig. 17 is a graph chart showing a correlation between a ratio X/R of a reactance to a resistance in a bioelectrical impedance when using an alternating current of the frequency of 50 kHz and a ratio $Z_5/Z_{250}$ of bioelectrical impedances when using an alternating current of the frequency of 5 kHz to a bioelectrical impedance when using an alternating current of the frequency of 250 kHz in a second embodiment of the present invention;

Fig. 18 is a flow chart showing a method for assessing condition in the second embodiment;

Fig. 19 is an explanatory view showing a condition information processing system in a third embodiment of the present invention;

Fig. 20 is a block diagram showing a detailed structure of a body composition analyzer in the third embodiment;

Fig. 21 is a block diagram showing a detailed structure of the condition information processing apparatus in the third embodiment;

Fig. 22 is a flow chart showing operations of the body composition analyzer in the third embodiment;

Fig. 23 is a flow chart showing operations of the condition information processing apparatus in the third embodiment;

Fig. 24 is an explanatory view showing a display example in the condition information processing apparatus in the third embodiment;

Fig. 25 is an explanatory view showing a display example in the condition information processing apparatus in the third embodiment;

Fig. 26 is an explanatory view showing a display example in the condition information processing apparatus in the third embodiment;

Fig. 27 is an explanatory view showing a display example in the condition information processing apparatus in the third embodiment;

Fig. 28 is an explanatory view showing a display example in the condition information processing apparatus in the third embodiment;

Fig. 29 is an explanatory view showing a condition information processing system in a fourth embodiment of the present invention;

Fig. 30 is a block diagram showing a detailed structure of the condition information processing apparatus in the fourth embodiment;

Fig. 31 is a block diagram showing a detailed structure of a terminal device in the fourth embodiment;

Fig. 32 is a flow chart showing operations of the condition information processing apparatus in the fourth embodiment;

Fig. 33 is a flow chart showing operations of the condition information processing apparatus in the fourth embodiment;

Fig. 34 is a flow chart showing operations of the terminal device in the fourth embodiment;

Fig. 35 is an explanatory view showing a condition information processing system in a fifth embodiment of the present invention;

Fig. 36 is a block diagram showing a detailed structure of the condition information processing apparatus in the fifth embodiment;

Fig. 37 is a flow chart showing operations of the condition information processing apparatus in the fifth embodiment;

Fig. 38 is a graph chart showing a correlation between a ratio X/R of a reactance to a resistance in a bioelectrical impedance when using an alternating current of the frequency of 50 kHz and a ratio $Z_{250}/Z_5$ of a bioelectrical impedance when using an alternating current of the frequency of 250 kHz to a bioelectrical impedance when using an alternating current of the frequency of 5 kHz in a variation of the present invention;

Fig. 39 is a graph chart showing a correlation between a ratio R/X of resistance to reactance in a bioelectrical impedance when using an alternating current of the frequency of 50 kHz and a ratio $Z_5/Z_{250}$ of a bioelectrical impedance when using an alternating current of the frequency of 5 kHz to a bioelectrical impedance when using an alternating current of the frequency of 250 kHz in a second embodiment of the present invention; and

Fig. 40 is a graph chart showing a correlation between a ratio R/X of resistance to reactance in a bioelectrical impedance when using an alternating current of the frequency of 50 kHz and a ratio $Z_{250}/Z_5$ of a bioelectrical impedance when using an alternating current of 250 kHz to a bioelectrical impedance when using an alternating current of the frequency of 5 kHz in a variation of the present invention.

DETAILED DESCRIPTION OF PREFERRED EMBODIMENTS

FIRST EMBODIMENT

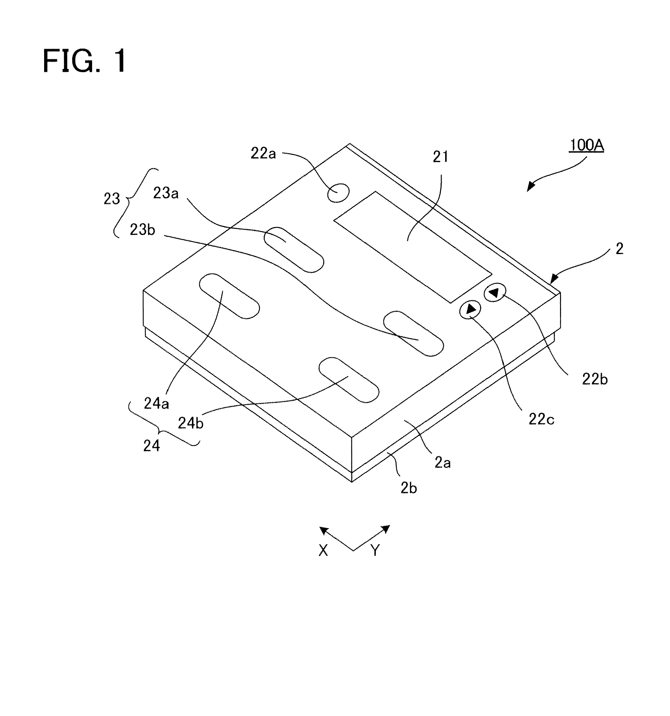

Referring to Figs. 1 to 16, the first embodiment of the present invention will be described below.

A. Structure

Fig. 1 is an outside view of a condition information processing apparatus 100A according to the embodiment. The condition information processing apparatus 100A according to the embodiment has functions of measuring information relating to obesity of a user such as a subcutaneous fat thickness, muscle mass, a body fat percentage and a body fat mass by well-known methods in addition to measuring weight and assessing the condition of the user.

As shown in Fig. 1, the condition information processing apparatus 100A includes a main unit 2 formed into almost a box-shape and a leg (not shown) provided at the reverse side of the main unit 2 to support the main unit 2. The main unit 2 is formed into almost a box-shape by combining a covering member 2a and a bottom board member 2b made by molding a resin (e.g., ABS resin (acrylonitrile-butadiene-styrene copolymer)) and the like. The main unit 2 may be formed by combining the covering member 2a, which is made of a resin as described above, and the bottom board member 2b, which is made of metal.

As shown in Fig. 1, a display section 21, an input section 22 (22a, 22b, 22c), and bioelectrical impedance measuring electrodes 23, 24 are provided on the surface of the covering member 2a of the main unit 2.

The bioelectrical impedance measuring electrodes 23, 24 are electrodes for measuring information relating to a bioelectrical impedance of the user by making contact with the skin of the user's sole. In the embodiment, the bioelectrical impedance measuring electrodes 23, 24 includes a pair of current applying electrodes 23 and a pair of voltage measuring electrodes 24, which are thin plate electrodes and arranged separately from each other on the surface of the covering member 2a. The current applying electrodes 23 includes a first current applying electrodes 23a and a second current applying electrodes 23b. The voltage measuring electrodes 24 includes a first voltage measuring electrode 24a and a second voltage measuring electrode 24b.

In the bioelectrical impedance measuring electrodes 23, 24, the first current applying electrodes 23a and the second current applying electrodes 23b are arranged at positions separately from each other in the X direction shown in Fig. 1. Furthermore, the first voltage measuring electrodes 24a and the second voltage measuring 24b are also arranged at positions separately from each other in the X direction. More specifically, the first current applying electrodes 23a is arranged at a position on which the user's left foot is placed. The second current applying electrodes 23b is arranged at a position on which the user's right foot is placed. The first voltage impressing electrodes 24a is arranged so that it is opposite to the first current applying electrodes 23a in the Y direction shown in Fig. 1 and located at a position on which the user's left foot is placed. The second voltage measuring electrodes 24b is arranged so that it is opposite to the second current applying electrode 23b and located at a position on which the user's right foot is placed.

Various types of structures for holding the bioelectrical impedance measuring electrodes 23, 24 can be appropriately selected. For example, it is preferable to form a concavity (not shown in Fig. 1), in which the bioelectrical impedance measuring electrodes 23, 24 can be embedded, on the covering member 2a to embed and hold them in the concavity so that they are flush with the surface of the covering member 2a of the main unit 2 as shown in Fig. 1.

As shown in Fig. 1, the display section 21 and the input section 22 are provided on the covering member 2a of the main unit 2 in addition to the bioelectrical impedance measuring electrodes 23, 24. The display section 21 displays data sent from a control section 31 provided in the inside of the main unit, so that it mainly displays various kinds of biological information of the user and a guide to operations, etc. A liquid crystal display such as a full-dot LCD can be utilized as the display section 21, for example.

The input section 22 is a section for inputting biological information of the user (e.g., gender, age, height) and for conducting various types of settings of the condition information processing apparatus 100A. In the embodiment, the input section 22 includes a setting key 22a, an up key 22b, and a down key 22c. The up key 22b and the down key 22b are used for selecting information and numerical value. The setting key 22a is used for setting the selected information and numerical value.

In the embodiment, various types of operating modes can be selected by operating the input section 22. The operating modes include a weight-measuring mode, a body composition-measuring mode, and a condition-estimating mode, for example. The weight-measuring mode is a mode for measuring the weight of the user. The body composition-measuring mode is a mode for measuring information such as a subcutaneous fat thickness, a muscle mass, a body fat percentage and a body fat mass of the user. The condition-estimating mode is a mode for assessing the condition of the user on the basis of the bioelectrical impedance of the user.

In the embodiment, the input section 22 including three button types of keys is provided in the side of the display section 21, for example. However, the number, form, and way of operation of the input section 21 are not limited to the example. A touch sensor type, or a dial type of the input section 21 can be utilized appropriately. A foot switch operable by the user's foot may be proved in the side of the main unit 2. The display section 21 and the input section 22 may be provided as a liquid crystal display panel having a touch panel function integrally. The biological information of the user and the setting item are stored in a memory section 34 and displayed on the display section 21.

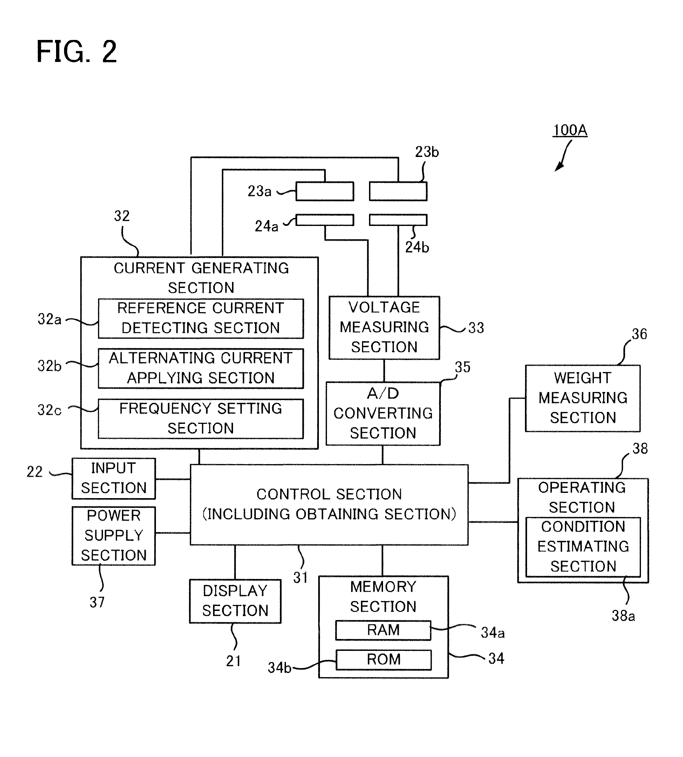

Fig. 2 is a block diagram showing the inside structure of the condition information processing apparatus 100A shown in Fig. 1 in a form of function block. The function block, which is provided of hardware such a device, a piece of equipment, software having the function thereof, or a combination thereof, indicates a function unit for achieving a predetermined operation.

As shown in Fig. 2, the condition information processing apparatus 100A includes a current generating section 32, a voltage measuring section 33, a weight measuring section 36, a power supply section 37, the memory section 35, a control section 31, an A/D converting section 35, and an operating section 38 in addition to the display section 21, the bioelectrical impedance measuring electrodes 23, 24, and the input section 22, which are described above.

The power supply section 37 supplies electrical power to each section of an electric power system of the condition information processing apparatus 100A. In the embodiment, a battery or an external power unit, which supplies electrical power to operate the condition information processing apparatus 100A, can be utilized as the power supply section 37. The control section 31, which is a processor such as a CPU or a DSP (Digital Signal Processor), is connected to each section electrically to control the operation of each section. In the embodiment, the control section 31 has a function of an obtaining section. The obtaining section of the embodiment has a function to obtain a voltage measured by the voltage measuring section 33.

The current generating section 32 generates an alternating current flowing between the first current applying electrode 23a and the second current applying electrode 23b when the user steps on the main unit 2 so that the user's feet make contact with the first current applying electrode 23a and the second current applying electrode 23b. The current generating section 32 includes a reference current detecting section 32a, an alternating current applying section 32b, and a frequency setting section 32c. The control section 31 controls the frequency setting section 32c to set a predetermined frequency. The frequency setting section 32c sets a low frequency of 5kHz and a high frequency of 250 kHz as frequencies of the alternating current impressed by the alternating current applying section 32b.

The reference current detecting section 32a detects a current flowing in the user to output a reference current detected signal to the alternating current applying section 32b. The alternating current applying section 32b generates an alternating current having a current value based on the reference current detected signal and the set frequency to impress the alternating current to the first current applying electrode 23a and the second current applying electrode 23b. The alternating current is impressed to the user through the first current applying electrode 23a and the second current applying electrode 23b.

In the embodiment, the frequency of the alternating current output from the current generating section 32 can be set to multiple values in accordance with the object of measurement. For example, upon assessing condition, the frequency of 5 kHz is set as the low frequency, and the frequency of 250 kHz is set as the high frequency.

The voltage measuring section 33 measures a voltage between the first voltage measuring electrode 24a and the second voltage measuring electrode 24b in order to calculate a bioelectrical impedance at the time of impressing the alternating current of 5 kHz as the low frequency and the alternating current of 250 kHz as the high frequency. A potential difference signal, which is measured in the voltage measuring section 33 as an analog signal, is converted to a digital signal in the A/D converting section 35 to be input to the control section 31.

The memory section 34 includes a RAM 34a and a ROM 34b, which stores data of various types of information, programs, and the like. The RAM 34a temporarily stores various types of measured data, the result of operation, and the like in addition to body specified information such as gender, height, age, which is input by the input section 22, and the subjective information relating to condition of the user's body.

In the ROM 34b, a program for controlling the apparatus, a program for calculating indexes relating to the predetermined estimation of condition to estimate the condition, frequencies of the alternating currents impressed upon measuring a voltage and the like. In the ROM 34b, the ratio of the bioelectrical impedance and condition scores as scores relating to condition, which are calculated by the operating section 38, are recorded as a measurement history together with additional information associated with each of the condition scores. The additional information includes body information such as weight, body temperature, blood pressure, when measuring the bioelectrical impedance, and objective information such as result information (e.g., times in a 100-meter dash, batting average) on the day when the measurement is performed. The additional information also includes subjective information such as information about whether or not the user suffers from injury or illness, text data indicating physical condition and feeling, and five grade evaluation data, which are input by the user's operation.

In the embodiment, injury caused by another person such as external injury and fracture due to a contact with another person is not included in the information about whether or not the user suffers from injury. Self-induced injury, such as a stress fracture due to excessive exercise, and pathological fracture, is included in the information about whether or not the user is suffering from injury.

The weight measuring section 36 measures weight placed on the main unit 2 to output a load signal. In the embodiment, the weight measuring section 36 includes a flexure element made from a metallic element deformed in accordance with the weight placed thereon, and a load cell consisting of a strain gauge attached to the flexure element. The main unit 2 is supported by one edge of the flexure element, another of which is supported by a leg (not shown). Thus, the flexure element is bent due to weight when the user steps on the surface of the main unit 2, so that the strain gauge expands and contracts. As a result, the value of resistance (the value of output) changes in accordance with the expansion and contraction of the strain gauge, so that the change of the resistance is measured as the change of the load signal. The load signal is converted to a digital signal by the A/D converter to be input in the control section 31.

The operating section 38 performs various kinds of arithmetic processing such as one in which weight is calculated on the basis of the A/D converted data of the load signal output from the weight measuring section 36, one in which a ratio of a bioelectrical impedance described below is calculated, one in which a condition score is calculated, one in which condition is estimated, and the like. In the embodiment, the operating section 38 includes a condition estimating section 38a.

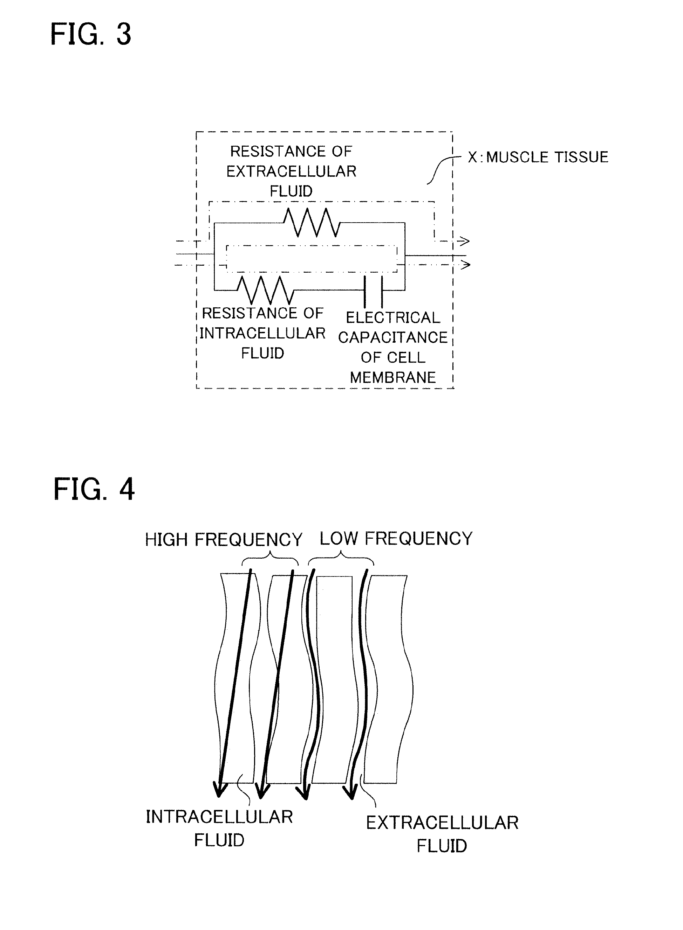

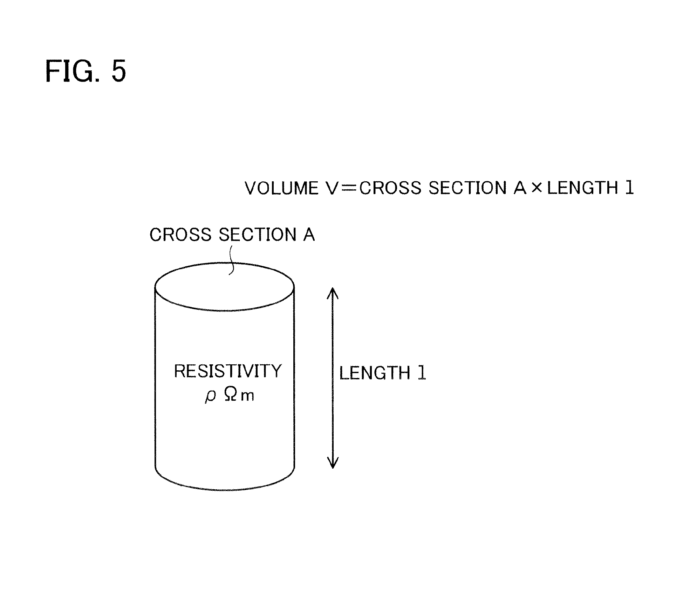

A detailed method of estimating the condition performed by the operating section 38 will be described below. Fig. 3 is a model diagram in which a current path of a living body in the embodiment is modeled by an electrical equivalent circuit. Fig. 4 is a model diagram showing a current path for each frequency in an electrolyte tissue mainly indicating a muscle tissue in the embodiment. Fig. 5 is a model diagram for explaining a relationship between a volume and a bioelectrical impedance in the embodiment.

The control section 31, the operating section 38, and the condition estimating section 38a are function blocks embodied by executing a program of the condition information processing apparatus in a CPU (not shown).

A bioelectrical impedance method will be described by referring to Figs. 3 to 5. In a biological tissue, most of lean tissues are body water including a large quantity of electrolytes, so that electric current is easy to flow. A fat tissue or a bone is considered as a non-electrolyte that includes very small quantities of electrolytes. Therefore, a muscle tissue, which is a lean tissue, is an electrolyte. Subcutaneous fat and visceral fat, which is fat tissue, is a non-electrolyte.

When the first current applying electrode 23 a and the second current applying electrode 23b are contacted to the body of the user, an electrical current flows through subcutaneous fat into a muscle tissue and the like, which has higher electrical capacity than a fat tissue. As shown in Fig. 3, a muscle tissue can be modeled as a circuit X. The circuit X indicates an electrical equivalent circuit of an electrolyte tissue, which is represented by a series circuit including a resistance of intracellular fluid and an electrical capacitance of cell membrane and a parallel circuit including a resistance of extracellular fluid.

The circuit X models an electrolyte tissue at a cellular level. As shown in Fig. 4, the electrolyte tissue includes a cell, in which the intracellular fluid is covered by the cell membrane, and the extracellular fluid (a connective tissue) exists at the outside of the cell. The intracellular fluid and the extracellular fluid function as a resistance. The cell membrane is considered to be an insulator. Since the cell membrane is formed by a lipid bilayer, it has electrical capacity. Therefore, the cell membrane becomes an insulator electrically when an alternating current has a frequency close to a direct current, so that an electrical current does not flow into the intracellular fluid. However, the electrical current flows into the intracellular fluid through the cell membrane in proportion as the frequency of the alternating current becomes higher, so that the electrical equivalent circuit can be described in that cell membrane as a condenser, and the intracellular fluid and the extracellular fluid are resistances.

In the model shown in Fig. 3, if a direct current is used, as shown with a dashed line, a resistance of the extracellular fluid is a current path, so that a measured value reflects the information of the extracellular fluid. If an alternating current is used, as shown by a two-dot chain line, the resistance of the extracellular fluid, the resistance of the intercellular fluid, and the electrical capacity of the cell membrane are current paths, so that the measured value reflects the information of the extracellular fluid and the intercellular fluid. The electrical capacity of the cell membrane has a smaller influence on the measured value in proportion as the frequency of the alternating current becomes higher, so that the measured value reflects the information of the resistance of the intercellular fluid to a greater extent. Therefore, a measured bioelectrical impedance reflects muscle cells to a greater extent in proportion as the frequency of the alternating current becomes higher.

Considering a model of substance having a volume V, a resistivity $\rho\Omega m$, cross section A, length l as shown in Fig. 5, the bioelectrical impedance Z is indicated by the following equation.

$$Z = \rho \cdot l / A \quad ----- (1)$$

Since the volume V is indicated by A·l, the volume V is indicated by the following equation.

$$V = A \cdot l = \rho \cdot l^2 / Z \quad ----- (2)$$

As described above, if an alternating current is impressed to a living body, the alternating current flows into the extracellular fluid in a low frequency range. If the value of the bioelectrical impedance calculated on the basis of the alternating current having a low frequency is applied to the equation (2), which is an equation of the volume, a value of the volume obtained by the equation (2) is a value of the volume of the extracellular fluid. If a value of the bioelectrical impedance measured by using the alternating current having a low frequency is $Z_L$, and a resistivity is $\rho_L$, a volume of the extracellular fluid $V_E$ is indicated as the following equation.

$$V_E = \rho_L \cdot l^2 / Z_L \quad ----- (3)$$

In a high frequency range, it is possible to ignore a condenser element of the cell membrane. Thus, if the value of the bioelectrical impedance calculated on the basis of the alternating current having a high frequency is applied to the equation (2), which is an equation of the volume, a value of the volume obtained by the equation (2) is a value of the volume of the entire tissue including the intracellular fluid. If a value of the bioelectrical impedance measured by using the alternating current having a high frequency is $Z_H$, and a resistivity is $\rho_H$, a volume Vw of the entirety of the tissue is indicated as the following equation.

$$V_W = \rho_H \cdot l^2 / Z_H \quad ----- (4)$$

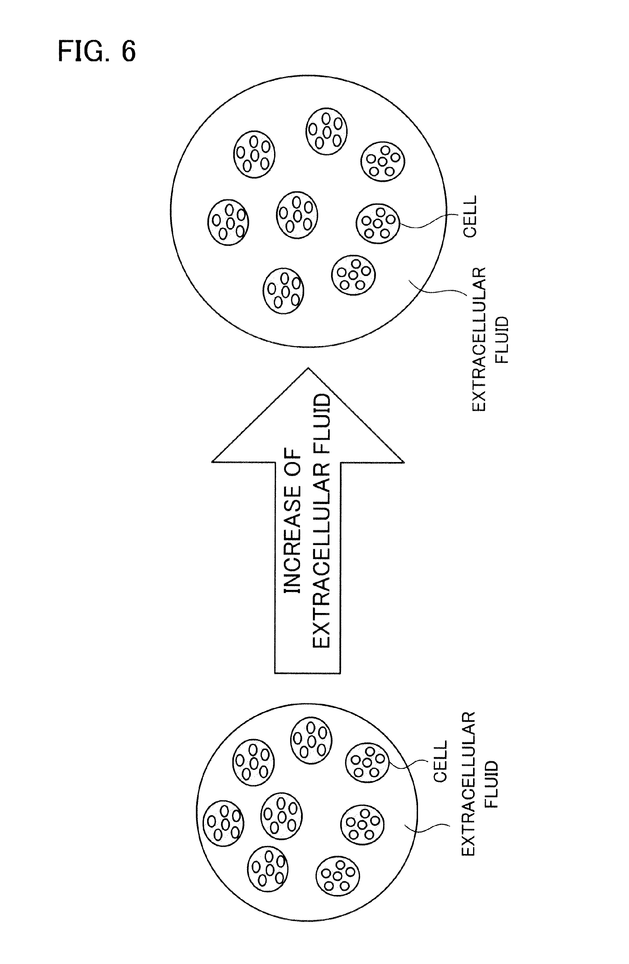

If the user gets injured, or develops disease, a certain tissue becomes inflamed or swollen. Therefore, the amount of blood, lymph, interstitial fluid and the like, which are included extracellularly, is increased, so that the amount of the extracellular fluid occupying a unit area is increased as may be understood from the section diagram shown in Fig. 6. A ratio $V_W/V_E$ of the volume of a cell in relation to the entirety of the tissue decreases. This is represented by using the equations (3) and (4) as follows.

$$V_W/V_E = (\rho_H \cdot l^2 / Z_H)/(\rho_L \cdot l^2 / Z_L) = (\rho_H \cdot Z_L)/(\rho_L \cdot Z_H) \quad ----- (5)$$

Assume that the resistivity $\rho_L$ calculated when the alternating current having a low frequency is almost the same as the resistivity $\rho_H$ calculated when the alternating current having a high frequency, the equation (5) is indicated as follows.

$$V_W/V_E = (\rho_H \cdot Z_L)/(\rho_L \cdot Z_H) \approx Z_L/Z_H \qquad \text{----- (6)}$$

where $0 < Z_H/Z_L < 1$

It is understood that the ratio $Z_L/Z_H$ of the value of the bioelectrical impedance calculated on the basis of the alternating current having a low frequency in relation to the value of the bioelectrical impedance calculated on the basis of the alternating current having a high frequency is the ratio of the extracellular in relation to a subject tissue. If swelling or inflammation worsens, the amount in the extracellular is increased. Therefore, it is considered that the ratio $Z_L/Z_H$ of the bioelectrical impedance indicated by the equation (6) approaches zero.

In the embodiment, if the body condition-estimating mode is selected, the operating section 38 calculates the ratio $Z_5/Z_{250}$ of the bioelectrical impedance $Z_5$ calculated by using the alternating current of low frequency of of 5 kHz to the bioelectrical impedance $Z_{250}$ calculated by using the alternating current of high frequency of 250 kHz. Specifically, the operating section 38 measures the bioelectrical impedance by switching the frequency of the alternating current impressed to the user between 250 kHz and 5 kHz to calculate the ratio $Z_5/Z_{250}$.

The operating section 38 calculates a condition score, which is an index relating to body condition, on the basis of the calculated ratio $Z_5/Z_{250}$ of the bioelectrical impedances. To calculate the condition score, in the embodiment, the operating section 38 sums up the ratio $Z_5/Z_{250}$ of the bioelectrical impedances relating to condition scores, which satisfy a predetermined condition, in the measurement history to calculate a deviation value of the most recently calculated ratio $Z_5/Z_{250}$ of the bioelectrical impedances. The equation for calculating this condition score Ti is represented as follows.

$$Ti = ((Xi - \mu x)/\sigma x) \cdot 10 + 50 \qquad \text{----- (7)}$$

where Xi is a calculated value, $\mu x$ is an average value, and $\sigma x$ is a standard deviation value.

The calculated value Xi is newly calculated ratio $Z_5/Z_{250}$ of the bioelectrical impedances. The average value $\mu x$ is the average of multiple ratios $Z_5/Z_{250}$ of the bioelectrical impedances, which are measured in the past and recorded as the measurement history. The standard deviation value $\sigma x$ is a value indicating dispersion of the multiple ratios $Z_5/Z_{250}$ of the bioelectrical impedances.

When calculating the average value $\mu x$ and the standard deviation value $\sigma x$, the operating section 38 has a function to appropriately select the objective information and the subjective information included in the additional information to aggregate the ratios $Z_5/Z_{250}$ of the bioelectrical impedances associated with condition scores, which satisfy the condition, in the measurement history. The operating section 38 can extract and narrow objective information and subjective information to select the objective information and the subjective information, which satisfy predetermined conditions, in the past measured values or to select the objective information and the subjective information in a predetermined period. In the embodiment, the operating section 38 selects the multiple ratios $Z_5/Z_{250}$ of the bioelectrical impedances, in which the user is in the condition that the user has no injury or disease, from the measurement history stored in the memory section 34. The operating section 38 calculates the average value $\mu x$ and the standard deviation value $\sigma x$ on the basis of these ratios $Z_5/Z_{250}$ of the bioelectrical impedances to store data in the ROM 34b, so that it reads out the data to calculate the condition score Ti.

The condition estimating section 38a estimates body condition on the basis of the condition score Ti, which is calculated on the basis of a newly calculated bioelectrical impedance, and the condition scores Tn, which are recorded as the measurement history. In the embodiment, the estimated condition and advice are displayed on the display section 21.

Specifically, if the value of the condition score Ti is lower than the condition score Tn, which is 50 points as the deviation value, included in the measurement history, it is estimated that the user will be exhausted so that the user's body is inflamed or swollen, so that the condition score may decline, or the user may be injured, or may develop disease. The message relating to this estimation is displayed on the display section 21.

For example, even if the calculated condition score is higher than 50 points, in the cases in which the condition score declines for a predetermined number of times (e.g., twice) than the previous value, it is estimated that there is a change of the condition, of which the user is unaware. The message, in which the condition is declining, so that the user should pay attention to it, is displayed on the display section 21.

The condition estimating section 38a has a function to estimate body condition by taking into account the additional information included in the measurement history. Specifically, for example, if the addition information, in which injury or disease is caused, is included in the previously calculated condition score Tn, and the newly calculated condition score is the same as or close to the condition score Tn, which is calculated at the time of suffering from injury or diseases, it is estimated that injury or disease may occur again, so that the message relating to the estimation is displayed by the display section 21.

For example, even if the value of the condition score is over 50 points, which is an average value, in the cases in which the subjective addition information indicating that the user was in poor condition, which is input by the user, and the value of the newly measured condition score Ti is the same as or close to the condition score Tn in the poor condition, the message, in which the condition was poor last time, is displayed in the display section 21.

For example, if the additional information of injury or disease is included in the previous measurement history, and the newly calculated condition score Ti is higher than before, it is estimated that the user is recovering from injury or disease, so that the message relating to the estimation is displayed in the display section 21. If the calculated condition score Ti is under 50 points, it is estimated that the user has not completely recovered from the injury or disease, so that it is necessary for the user to pay attention to this. Then, the message relating to the estimation is displayed in the display section 21. The control section 31 has another function to calculate subcutaneous fat thickness, weight, muscle mass, body fat percentage, somatic fat volume, and the like.

B: Method for estimating condition

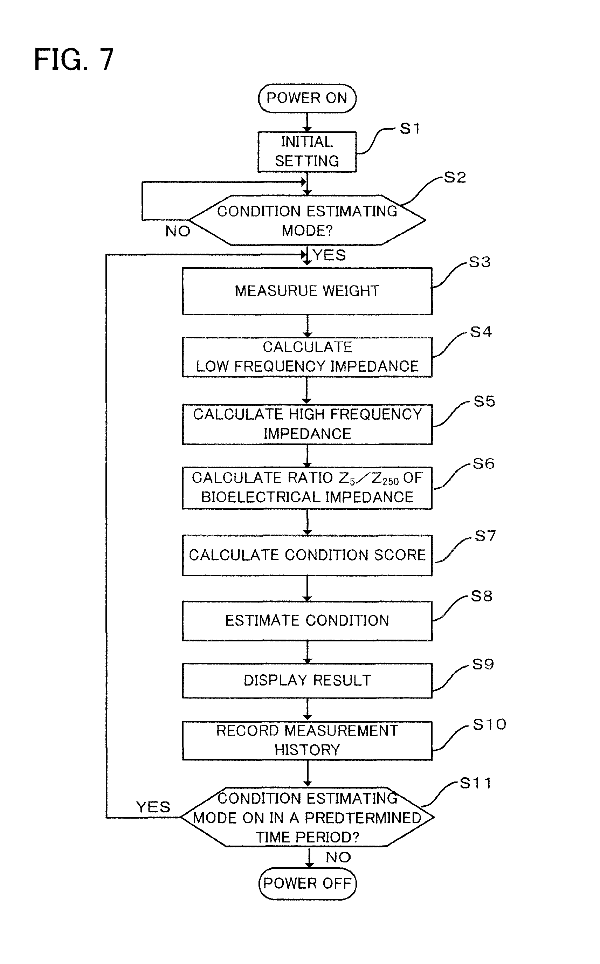

The operation of the condition information processing apparatus 100A will be described by referring to the flow chart shown in Fig. 7. Fig. 7 is a flow chart showing the method for estimating condition of the embodiment.

When the input section 22 is operated, so that the power of the condition information processing 100A is turned on, an initial setting of the apparatus is performed. Furthermore, a message for requesting the user to select an operation mode is displayed in the display section 21 by the instruction of the control section 31 (Step S1). The user can select the operation mode by using the setting key 22, the up key 22b, and the down key 22c.

The control section 31 determines whether or not an estimation mode of the body condition is selected as the operation mode (Step S2), so that if another mode other than the condition-estimating mode of the body condition is selected (Step S2:NO), it executes the processing of each operation mode. In the flow chart shown in Fig. 7, to simplify the explanation, it is described that the control section 31 is in a wait condition until the condition-estimating mode of the body condition is selected.

When the condition-estimating mode of the body condition is selected by the user (Step S2:YES), and the user steps on the main unit 2 to contact the user's body surface to the bioelectrical impedance measuring electrodes 23, 24, the user's weight is calculated on the basis of the load signal by the operating section 38 (Step S3). The low frequency of 5 kHz is set by the frequency setting section 32c. The alternating current of 5 kHz is impressed to the first current applying electrode 23a and the second current applying electrode 23b through the reference current detecting section 32a by the alternating current applying section 32b. The voltage between the first voltage measuring electrode 24a and the second voltage measuring electrode 24b is measured by the voltage measuring section 33. The measured voltage is converted to digital signals by the A/D converting section 35. The operating section 38 calculates the bioelectric impedance on the basis of the digital signal corresponding to the voltage and the reference current impressed by the alternating current applying section 32b (Step S4).

When the calculation of the bioelectrical impedance at the time of impressing the alternating current of 5 kHz is finished, as in the case of the low frequency, the high frequency of 250 kHz is set by the frequency setting section 32c. The alternating current of 250 kHz is impressed to the first current applying electrode 23a and the second current applying electrode 23b through the referenced current detecting section 32a by the alternating current applying section 32b. The voltage between the first voltage measuring electrode 24a and the second voltage measuring electrode 24b is measured by the voltage measuring section 33. The measured voltage is converted to digital signal by the A/D converting section 35. The operating section 38 calculates the bioelectric impedance on the basis of the digital signal corresponding to the voltage and the reference current impressed by the alternating current applying section 32b (Step S5).

When the calculation of the bioelectrical impedance $Z_5$ at the time of impressing the alternating current of 5 kHz and the bioelectrical impedance $Z_{250}$ at the time of impressing the alternating current of 250 kHz is finished, the ratio $Z_5/Z_{250}$ of the bioelectrical impedances is calculated by the operating section 38 (Step S6).

When the ratio $Z_5/Z_{250}$ of the bioelectrical impedances is calculated, the condition score Ti relating to the body condition on the basis of the calculated ratio $Z_5/Z_{250}$ of the bioelectrical impedances by the operating section 38 (Step S7).

Specifically, the operating section 38 aggregates the ratios $Z_5/Z_{250}$ of the bioelectrical impedances, which satisfy predetermined conditions, among the ratios $Z_5/Z_{250}$ of the bioelectrical impedances associated with the condition scores recorded as the measurement history by referring to the additional information stored in the memory section 34. The operating section 38 calculates the average value $\mu x$ and the standard deviation value $\sigma x$ in order to calculate the deviation value of the calculated ratio $Z_5/Z_{250}$ of the bioelectrical impedances. In the embodiment, for example, the condition, in which the user measures the bioelectrical impedance without injury or disease, is set as the predetermined condition. The operating section 38 appropriately selects the objective information or the subjective information included in the additional information recorded as the measurement history to aggregate the multiple ratios $Z_5/Z_{250}$ of the bioe-

lectrical impedances under the condition without injury or disease. Then, the operating section 38 calculates the average value $\mu x$ and the standard deviation value $\sigma x$ on the basis of those data.

The operating section 38 calculates the condition score Ti by using the equation (7) on the basis of the newly calculated value Xi of the ratio $Z_5/Z_{250}$ of the bioelectrical impedances, the average value $\mu x$ and the standard deviation value $\sigma x$. If the amount of the measured data recorded as the measurement history is small, the calculation of the condition score is not executed. When the condition score Ti is calculated, the condition estimating section 38a estimates the body condition on the basis of the newly calculated condition score Ti and the previous condition scores Tn recoded as the measurement history (Step S8). Specific examples for estimating the condition will be described later.

When the estimation of the body condition is finished, the value of the condition score Ti and the result of the estimation of the condition are displayed on the display section 21 (Step S9). The control section 31 stores the calculated condition score Ti together with the additional information as the measurement history in the memory section 34 (Step S 10).

After that, a message for asking the user whether or not the user selects the condition-estimating mode of the body condition again is displayed on the display section 21 in a predetermined period by the instruction of the control section 31 (Step S11). If the condition-estimating mode of the body condition is selected by the user (Step S11; YES), the processing starting from the measurement of the weight is repeated (Steps S3 to S10). If the condition-estimating mode of the body condition is not selected in a predetermined period (Step S11:NO), the control section 31 turns off the power to finish the processing.

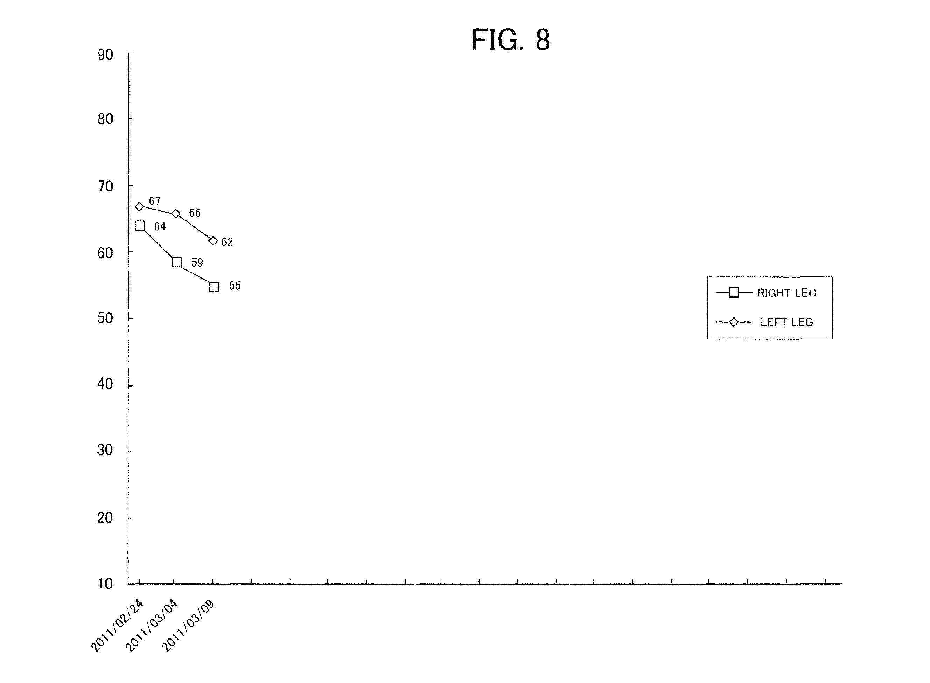

Specific examples of the estimation of the condition will be described below. Fig. 8 is a diagram for explaining an example in which the calculated condition score Ti is higher than 50 points, but it continuously declines compared to previous values two or more times. If the newly calculated condition score Ti is higher than 50 points, but it continuously declines compared to previous values two or more times, it is estimated that there is a change of condition, such as inflammation or swelling, of which the user is unaware, and there is a possibility of injury or disease. Thus, in the embodiment, the message, "Score is declining continuously. Are you so tired or in a poor condition? How about checking your body, or resting ?" is displayed.

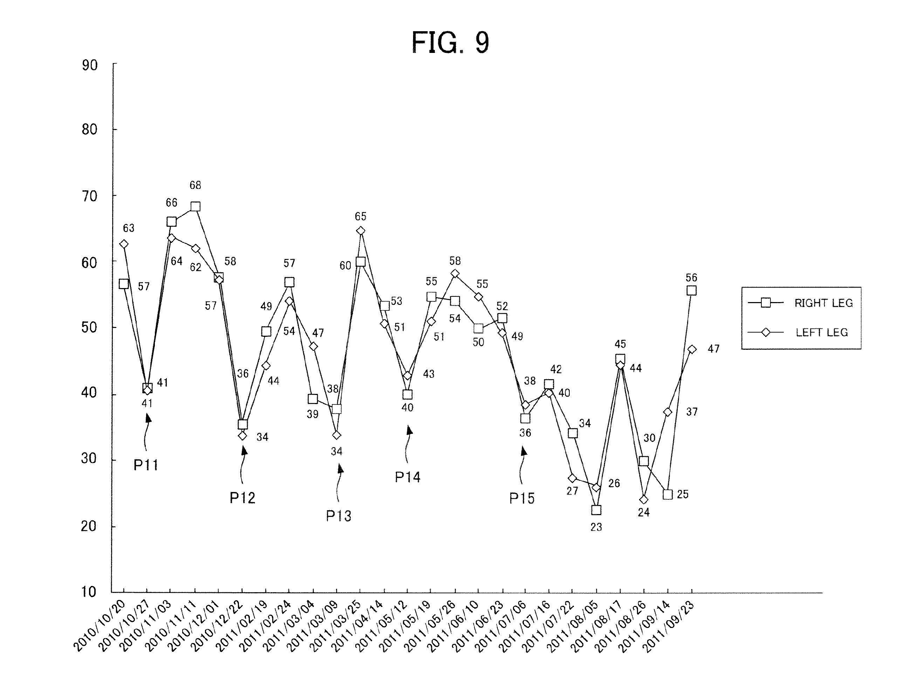

Fig. 9 is a diagram for explaining an example in which the calculated condition score Ti is lower than 50 points (P11 to P15). If the condition score Ti is lower than 50 points, it is anticipated that the condition score Ti will continue to be low. It is estimated that the user is so exhausted in the cases in which the condition score Ti has changed in this way. Thus, in the embodiment, the message, "Score declined lower than 50 points. Are you so exhausted or in a poor condition? How about checking your body or resting?" is displayed on the display section 21.

Since it is considered that the condition score Ti could increase in the future even if the calculated condition score Ti is lower than 50 points, the message, "Score continuously declines to less than 50 points. Are you so tired or in a poor condition? How about checking your body or resting?" may be displayed if the condition score Ti continuously declines to less than 50 points two or more times.

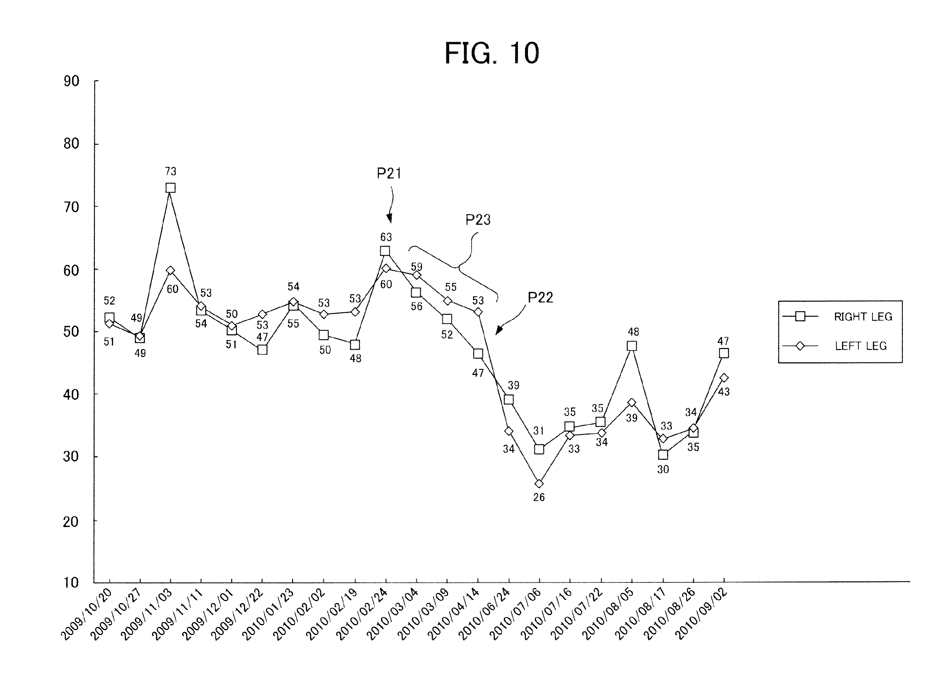

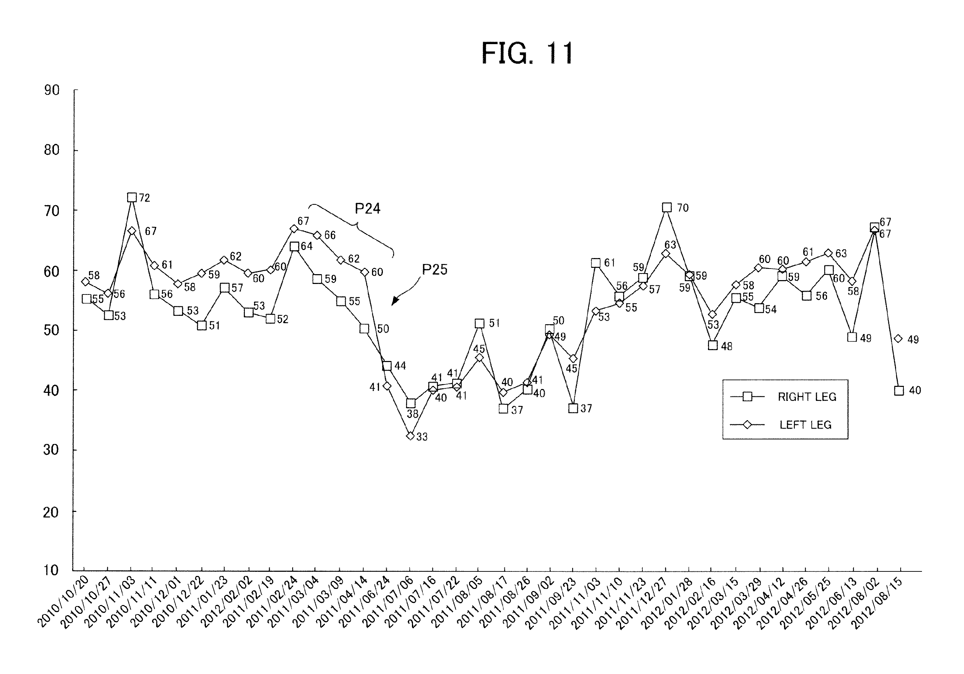

Fig. 10 is a diagram for explaining an example in which the condition score Ti begins to decline gradually from the condition score Ti on February 24, 2010 (P21), the condition score Ti continuously declines from March 4, 2010, to April 14, 2010 (P23), and the user has had an anterior cruciate ligament injury during a game around June 20, 2010 (P22). In that type of case, in the embodiment, the pattern of the change of the condition score Ti and the condition score Ti at the time of injury are stored. If the pattern, which is the same as the stored pattern of the change of the condition score Ti, appears, and the condition score Ti, which is close to the pattern at the time of injury, a warning message is displayed. An example, in which that the kind of warning message is displayed, will be described by referring to Fig. 11.

Fig. 11 is a diagram for explaining an example in which the pattern (P24) of the change of the condition score Ti from February 24, 2011, to April 14, 2011, indicates similar tendency for the stored pattern (P21, P23 in Fig. 10) of the change of the condition score Ti from February 24, 2010, to April 14, 2010. In this example, the condition score Ti (P25 in Fig. 10) calculated around April 24, 2011 is close to the condition score Ti (P22 in Fig. 10) at the time of the anterior cruciate ligament injury. In the embodiment, a warning message, "Score is close to the value at the time of injury on June 21, 2010. Are you in a poor condition or do you feel any pain? In order to prevent the recurrence of injury, it is recommended to rest." is displayed. Thus, if the user seeing this message takes a rest, or decreases the amount of practice, it is possible to prevent of the reoccurrence of injury and disease.

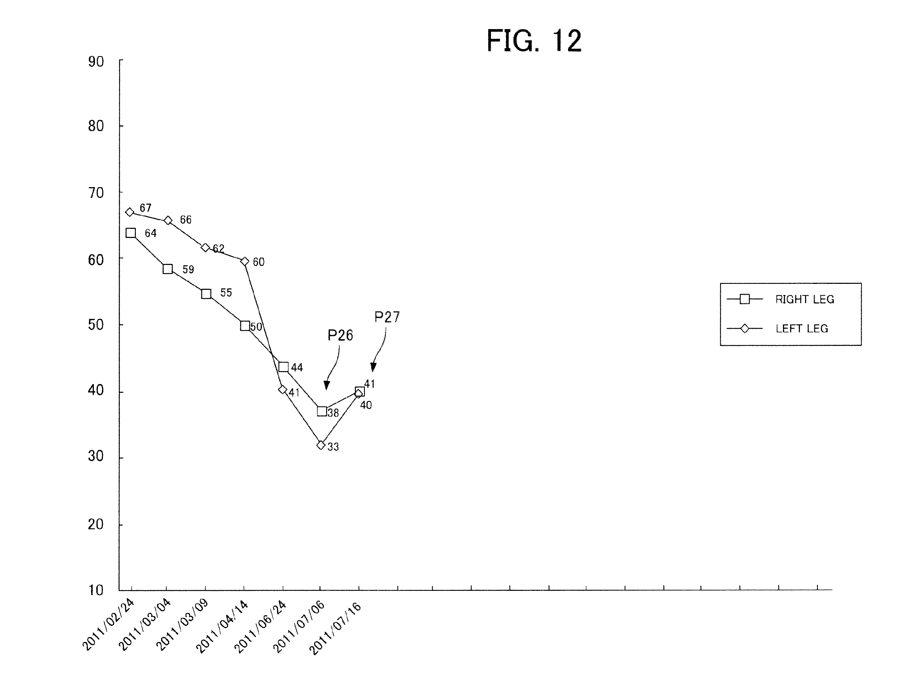

Fig. 12 is a diagram for explaining an example in which the condition score Ti (P26) calculated on July 6, 2011, falls below 40 points, so that the condition score Ti (P27) calculated on July 16, 2011, exceeds the last score. In this example, the message, "Score recovers the last point. Keep measuring body composition and observing progress." is displayed.

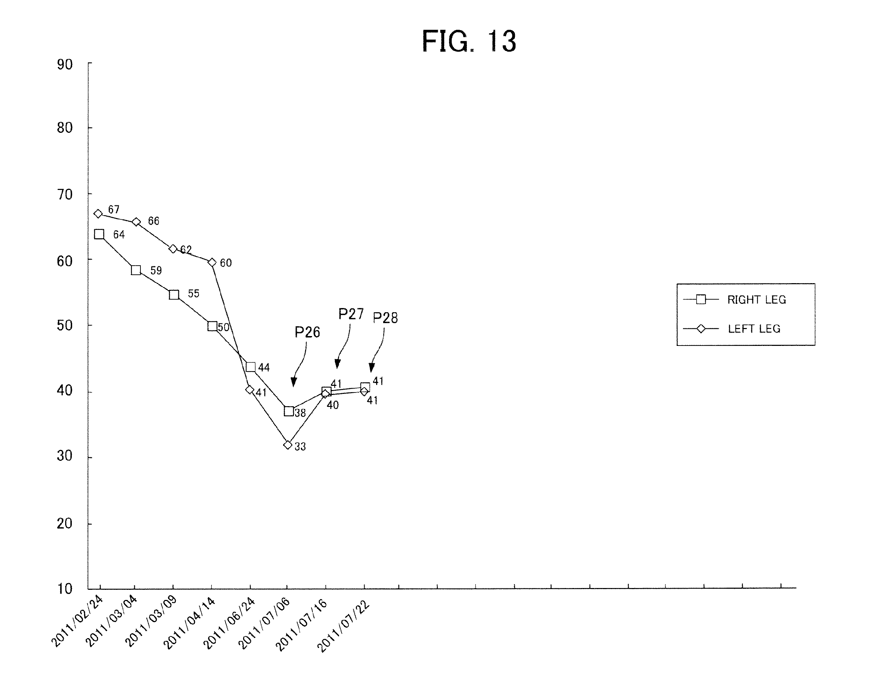

Fig. 13 is a diagram for explaining an example in which the condition score Ti recovers from the condition shown in Fig. 12, so that the condition score Ti (P28) calculated on July 22, 2011 exceeds the last score, namely, the condition score Ti continuously rises two or more times. In this case, the message, "Score is continuously rising. Keep measuring body composition and observing progress." is displayed.

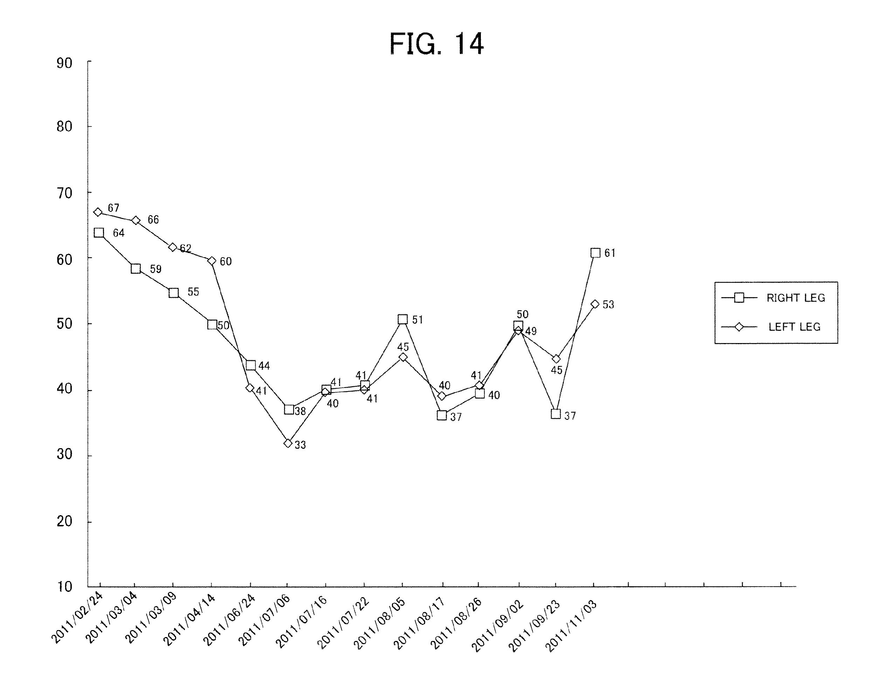

Fig. 14 is a diagram for explaining an example in which the condition score Ti is recovering from the condition shown in Fig. 13, so that it is over 50 points. In this case, the message, "Score is over 50 points. In order to increase the score even more, keep maintaining yourself physically and measuring to observe progress." is displayed.

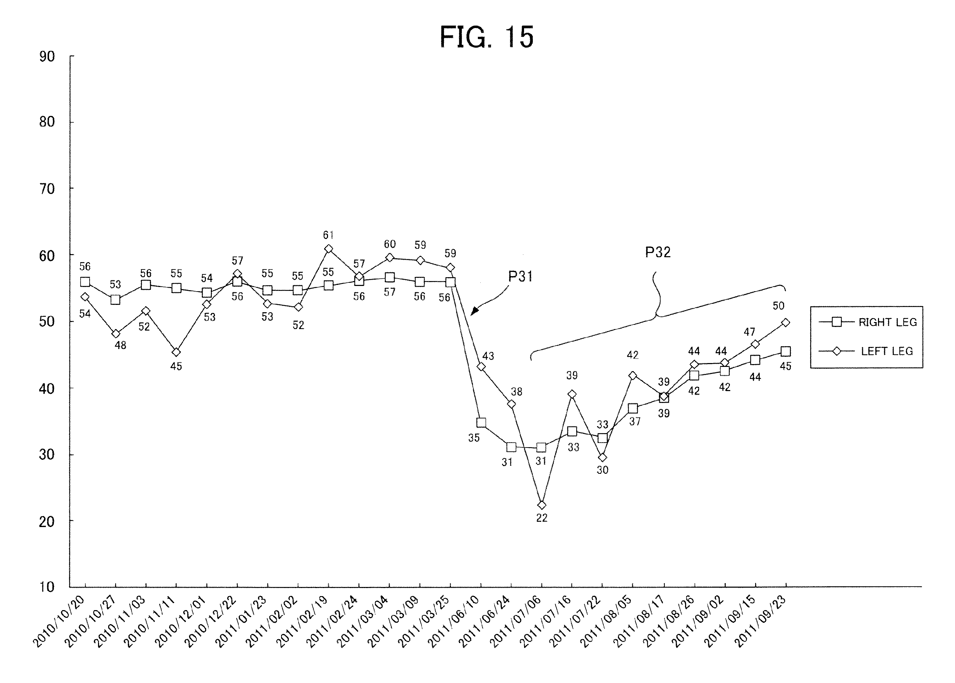

Fig. 15 is a diagram for explaining an example in which the user suffered a fracture during a game on April 10, 2011 (P31), so that the user entered a hospital and undergoes rehabilitation after leaving the hospital (P32). In this example, if the calculated condition score Ti is less than 50 points (P32), it is estimated that the user has not completely recovered from the injury, so that it is necessary for the user to pay attention to it. Then, the message, "Score is continuously recovering. Score is still less than 50 points. Be careful about overworking and keep maintaining yourself physically. Keep measuring body composition to observe progress.", is displayed on the display section 21. Thus, the user can know that the user is steadily recovering from the injury. The user's motivation will be increased. Since the user's attention to overwork is drawn, it is possible to prevent overwork before the user has not completely recovered from the injury and to prevent the recurrence of the injury.

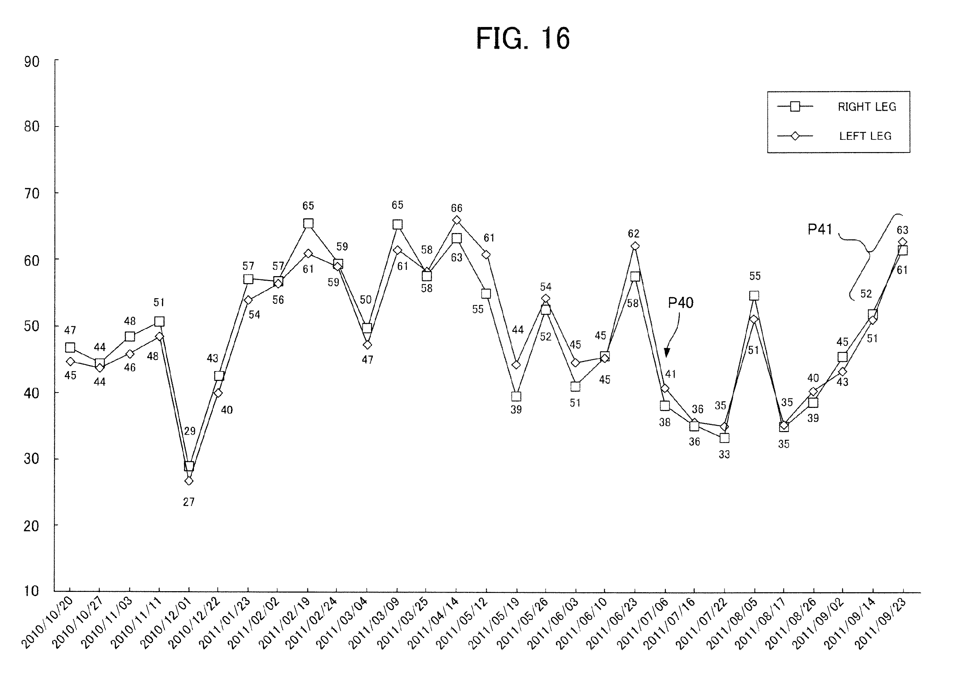

Fig. 16 is a diagram for explaining an example in which the user was injured around on July 10, 2011 (P40), which is stored in the apparatus, and the condition score Ti is continuously increasing to exceed 50 points (P41). In this case, since it is expected that the user has completely recovered from the injury, and the condition is good, the message, "Score is over 50 points. You seem to have completely recovered from the injury. In order to increase the score even more, keep maintaining yourself physically and measuring body composition to observe progress." is displayed on the display section 21. According to the embodiment, the user can know that the user has completely recovered from the injury by displaying the message of the complete recovery from the injury.

As described above, according to the embodiment, the bioelectrical impedance $Z_{250}$ at the time of impressing the alternating current of 250 kHz and the bioelectrical impedance $Z_5$ at the time of impressing the alternating current of 5 kHz are calculated. The ratio $Z_5/Z_{250}$ of the bioelectrical impedances is calculated. Then, the condition score Ti, in which the body condition is indicated as the deviation value on the basis of the ratio $Z_5/Z_{250}$ of the bioelectrical impedances, is calculated. Therefore, it is possible to objectively estimate the change of the ratio of the extracellular fluid such as inflammation and swelling, which is caused by injury or disease, for example, by the condition score Ti. It is possible to estimate the condition non-invasively and easily.

According to the embodiment, the recorded additional information associated with the condition score as the measurement history is referred to. Then, the condition score Ti is calculated by aggregating the ratios $Z_5/Z_{250}$ of the bioelectrical impedances, which satisfy a predetermined condition. For example, it is possible to calculate the deviation value, that is, the condition score Ti under the good condition by aggregating only the ratios $Z_5/Z_{250}$ of the bioelectrical impedances without injury or disease. It is possible to estimate the condition on the basis of the calculated condition score Ti and the past condition score recorded as the measurement history. Thus, if the calculated condition score is less than 50 points, it is possible to easily judge that the user's condition is poor.

In the embodiment, since the body condition is estimated on the basis of the newly calculated condition score Ti and the condition scores Tn recorded as the measurement score, it is possible to easily understand a recovery condition from the injury.

According to the embodiment, the condition score under the condition of injury or disease is referred to on the basis of the recorded additional information associated with the condition score as the measurement history. Then, the newly calculated condition score Ti is compared to the referred condition score. If the newly calculated condition score Ti rise up than the referred condition score, it is estimated that the user is recovering from injury or disease, so that it is possible to display the message of recovering from injury or disease.

SECOND EMBODIMENT

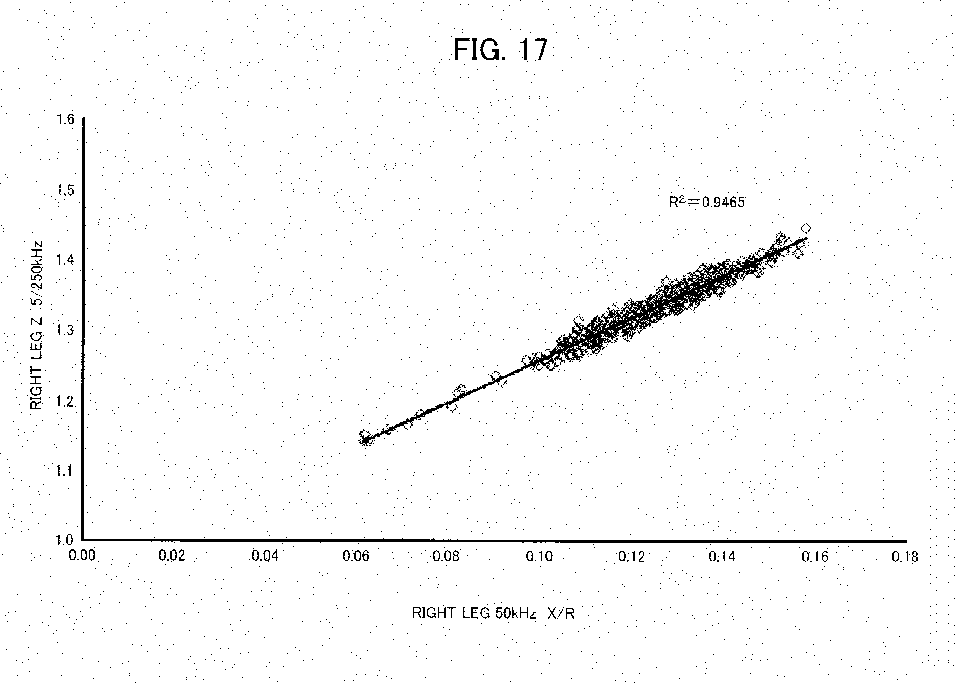

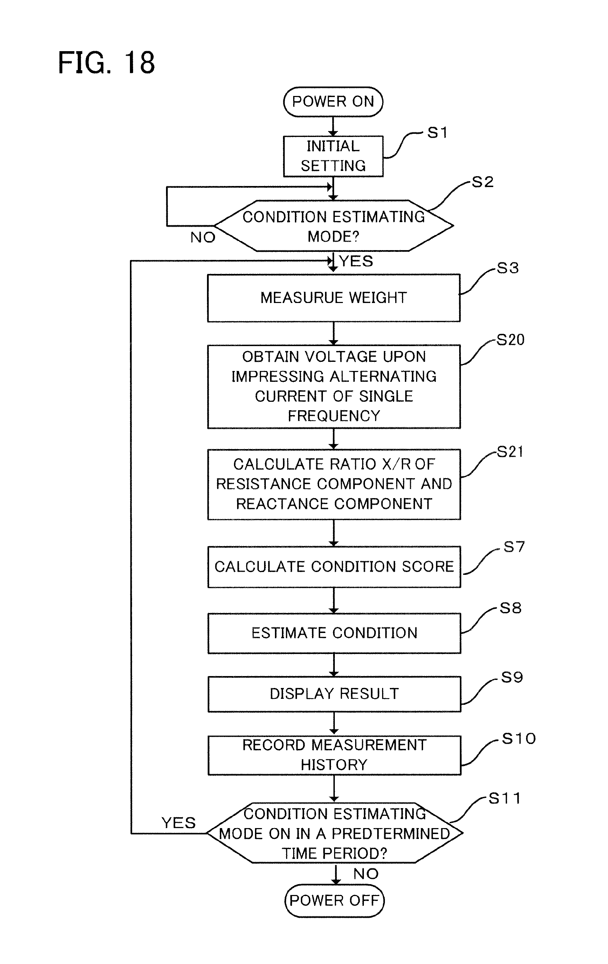

Referring to Fig. 17 and Fig. 18, the second embodiment of the present invention will be described below. The second embodiment is different from the first embodiment in the respect that the condition score is calculated on the basis of the ratio of a reactance component and a resistance component in the bioelectrical impedance instead of the ratio $Z_5/Z_{250}$ of the bioelectrical impedances. The second embodiment is also different from the first embodiment in the respect that the frequency of the alternating current, which is impressed to the user, is a single frequency.

In the first embodiment, the body condition is estimated by using the high frequency of 250 kHz and the low frequency of 5 kHz as the frequencies of the alternating current, which is impressed to the user. However, the present invention is not limited to this kind of example. As shown in Fig. 17, there is a correlation between the ratio $Z_5/Z_{250}$ of the bioelectrical impedances and the ratio X/R of a reactance component X and a resistance component R in the bioelectrical impedance at the time of impressing the alternating current of the frequency of 50 kHz, in which R is a resistance component in the calculated bioelectrical impedance, and X is a reactance component in the calculated bioelectrical impedance. Since there is a correlation between the ratio $Z_5/Z_{250}$ of the bioelectrical impedances and the ratio X/R of a reactance component X and a resistance component R, it is possible to calculate the condition score even if the ratio X/R of a reactance component X and a resistance component R is used instead of the ratio $Z_5/Z_{250}$ of the

bioelectrical impedances.

In the second embodiment, the alternating current of the single frequency of 50 kHz is impressed to the user, so that the ratio X/R of a reactance component X and a resistance component R in the bioelectrical impedance at the time of impressing the alternating current at a single frequency of 50 kHz is calculated. Then, the condition of the user is estimated on the basis of the ratio X/R.

In the second embodiment, if the estimating mode of the body condition is selected, the operating section 38 calculates the ratio X/R of a reactance component X and a resistance component R in the bioelectrical impedance at the time of impressing the alternating current the single frequency of 50 kHz. The operating section 38 calculates the condition score as an index of the body condition on the basis of the calculated ratio X/R of a reactance component X and a resistance component R, and the aggregated X/R of a reactance component X and a resistance component R.

When calculating the condition score in the second embodiment, the ratio X/R of the reactance component X and the resistance component R, which satisfies predetermined conditions, is aggregated among the past ratios X/R of the reactance components X and the resistance components R, which are recorded as the measurement history. Then, a deviation value of the newly calculated ratio X/R of the reactance component X and the resistance component R is calculated on the basis of the aggregated ratios X/R of the reactance components X and the resistance components R. The equation for calculating the condition score Ti is as follows.

$$Ti = ((Xi - \mu x)/\sigma x) \cdot 10 + 50 \qquad ----- (8)$$

where Xi is a calculated value, $\mu x$ is an average value, and $\sigma x$ is a standard deviation value.

The calculated value Xi is newly calculated ratio X/R of a reactance component X and a resistance component R. The average value $\mu x$ is the average of multiple ratios ratio X/R of a reactance component X and a resistance component R., which are calculated in the past. The standard deviation value $\sigma x$ is a value indicating dispersion of the multiple ratios X/R of a reactance component X and a resistance component R.

Since it is necessary to aggregate the ratios X/R of the reactance components X and the resistance components R, which satisfy predetermined conditions, for the calculation of the condition score, if the amount of data recorded as the measurement history is small, the ratio X/R of the reactance component X and the resistance component R is displayed on the display section 21 as it is.

The flow chart of the operation of the condition estimating apparatus 100A of the second embodiment will be described is Fig. 18. As shown in Fig. 18, when the input section 22 is operated, so that the power of the condition estimating apparatus 100A is turned on, an initial setting of the apparatus is performed. Furthermore, a message for requesting the user to select an operation mode is displayed in the display section 21 by the instruction of the control section 31 (Step S1). The user can select the operation mode by using the setting key 22, the up key 22b, and the down key 22c.

The control section 31 determines whether or not the estimating mode of the body condition is selected as the operation mode (Step S2), so that if another mode other than the estimation mode of the body condition is selected, it executes the processing of each operation mode. In the flow chart shown in Fig. 18, to simplify the explanation, it is described that the control section 31 is in a wait condition until the estimating mode of the body condition is selected.

When the estimating mode of the body condition is selected by the user (Step S2:YES), and the user steps on the main unit 2 to contact the user's body surface to the bioelectrical impedance measuring electrodes 23, 24, the user's weight is calculated on the basis of the load signal by the operating section 38 (Step S3). The frequency of 50 kHz is set by the frequency setting section 32c. The alternating current of 50 kHz is impressed to the first current applying electrode 23a and the second current applying electrode 23b through the reference current detecting section 32a by the alternating current applying section 32b. The voltage between the first voltage measuring electrode 24a and the second voltage measuring electrode 24b is measured by the voltage measuring section 33. The measured voltage is converted to digital signal by the A/D converting section 35. The operating section 38 obtains the digital signal (Step S20).

When the voltage at the time of impressing the alternating current of 50 kHz is obtained, the operating section 38 executes a waveform processing such as a DFT (Discrete Fourier Transform) by using the obtained voltage and the reference current detected by the reference current detecting section 32a, so that the operating section 38 obtains a reactance component X and a resistance component R. Then, the operating section 38 calculates the ratio X/R of a reactance component X and a resistance component R. (Step S21).

When the ratio X/R of the reactance component X and the resistance component R is calculated, the operating section 38 calculates the condition score Ti relating to the body condition on the basis of the calculated X/R of the reactance component X and the resistance component R (Step S7).

Specifically, the operating section 38 refers to the additional information, which is associated with the ratio X/R and is recorded as the measurement history in the memory section 34, so that the operating section 38 aggregates the ratios X/R of the reactance components X and the resistance components R, which satisfy predetermined condition. Then, the operating section 38 calculates the average value $\mu x$ and the standard deviation value $\sigma x$ in order to calculate the deviation value of the calculated ratio X/R of the reactance component X and the resistance component R. The operating section 38 appropriately selects the objective information or the subjective information included in the additional information recorded as the measurement history to aggregate the multiple ratios X/R of the reactance components X and the resistance components R under the condition without injury or disease. Then, the operating section 38 calculates the average value $\mu x$ and the standard deviation value $\sigma x$ on the basis of these data.

The operating section 38 calculates the condition score Ti by using the equation (8) on the basis of the newly calculated value Xi of the ratio X/R of the reactance component X and the resistance component R, the average value $\mu x$ and the standard deviation value $\sigma x$. If the amount of the measured data recorded as the measurement history is small, the calculation of the condition score is not executed.

When the condition score Ti is calculated, the condition estimating section 38a estimates the body condition on the basis of the newly calculated condition score Ti and the previous condition score Tn recoded as the measurement history (Step S8).

When the estimation of the body condition is finished, the value of the condition score Ti and the result of the estimation of the condition are displayed on the display section 21 by the instruction of the control section 31 (Step S9). The control section 31 stores the calculated condition score Ti together with the additional information as the measurement history in the memory section 34 (Step S10). If the amount of the measured data recorded as the measurement history is small, the calculated ratio X/R of the reactance component X and the resistance component R, and the calculated weight are displayed on the display section 21 as they are.

After that, a message for asking the user whether or not the user selects the estimating mode of the body condition again is displayed on the display section 21 in a predetermined period by the instruction of the control section 31 (Step S11). If the estimating mode of the body condition is selected by the user (Step S11:YES), the processing starting from the measurement of the weight is repeated (Steps S3 to S10). If the estimating mode of the body condition is not selected in a predetermined period (Step S11:NO), the control section 31 turns off the power to finish the processing.

According to the second embodiment, even if the alternating current of the single frequency as the alternating current impressed to the user is used, the condition score Ti, in which the body condition is indicated as the deviation value on the basis of the ratio X/R of the reactance component X and the resistance component R, is calculated. Therefore, it is possible to objectively estimate the change of the ratio of the extracellular fluid such as inflammation or swelling, which is caused by the injury or disease, for example, by the condition score Ti. It is possible to estimate the condition noninvasively and easily.


THIRD EMBODIMENT

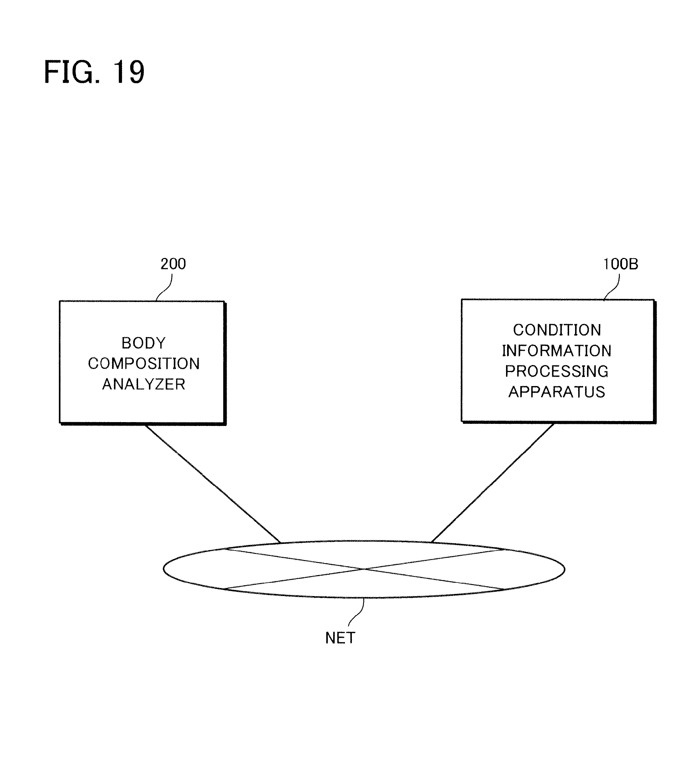

Referring to Figs. 19 to 27, the third embodiment of the present invention will be described below. Fig. 19 is a diagram showing a structure of the condition information processing system of the third embodiment of the present invention.

In the first embodiment, it is described that one apparatus executes the measurement of the weight, the measurement of the information relating to the bioelectrical impedance, the calculation of the ratio of the bioelectrical impedance, the calculation of the condition score, and the estimation of the condition.

However, the third embodiment is different from the first embodiment in the respect that the measurement of the weight and the measurement of the information relating to the bioelectrical impedance are executed by a body composition analyzer 200, and the calculation of the ratio of the bioelectrical impedance, the calculation of the condition score, and the estimation of the condition are executed by the condition information processing apparatus 100B.

In the third embodiment, the alternating current of low frequency of 5 kHz and the alternating current of high frequency of 250 kHz are used as the alternating current impressed to the user.

As shown in Fig. 19, the condition information processing system of the third embodiment includes the body composition analyzer 200 and the condition information processing apparatus 100B. The body composition analyzer 200 is an apparatus in which the operating function to calculate the ratio of the bioelectrical impedances, the operating function to calculate the condition score, and the function to estimate the condition are excluded from the condition information processing apparatus 100A of the first embodiment.

The body composition analyzer 200 of the third embodiment executes the measurement processing of the weight of the user and the measurement processing of the bioelectrical impedance as information relating to the bioelectrical impedance.

The condition information processing apparatus 100B of the third embodiment executes the calculation of the ratio of the bioelectrical impedance, the calculation of the condition score, and the estimation of the condition.

The condition information processing apparatus 100B is a personal computer, or a mobile terminal apparatus such as a smart phone, which is capable of communicating with the body composition analyzer 200 through a communication network NET such as the Internet or a LAN. The condition information processing apparatus 100B may communicate with composition analyzer 200 through a USB (Universal Serial Bus) cable, or Bluetooth, etc., other than the communication network NET. The condition information processing apparatus 100B has functions to calculate the ratio of the bioelectrical impedances on the basis of the bioelectrical impedance received from the body composition analyzer 200, to calculate the condition score on the basis of the ratio of the bioelectrical impedances, to estimate the condition, to show the change of the condition score as the result of the estimation graphically, and to show advice on the condition and the like, by installing a program for the condition information processing apparatus and executing the program.

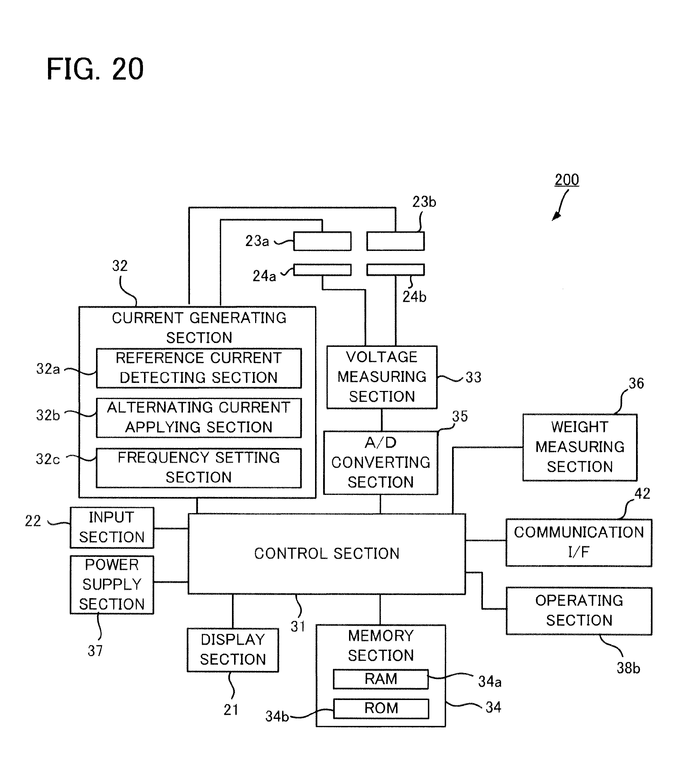

Fig. 20 is a block diagram showing an internal structure of the body composition analyzer 200 shown in Fig. 19. Each functional section is configured of hardware such as a device, equipment, software having functions, or combination thereof, so that it indicates a functional unit for achieving a predetermined operation.

As shown in Fig. 20, the body composition analyzer 200 includes a display section 21, an input section 22, bioelectrical impedance measuring electrodes 23, 24, a control section 31, a current generating section 32, a voltage measuring section 33, a memory section 34, an A/D converting section 35, a weight measuring section 36, a power supply section 37, an operating section 38b and a communication interface 42.

The operating section 38b of the body composition analyzer 200 in the embodiment executes the measurement processing of weight on the basis of the load signal measured by the weight measuring section 36, and the measurement processing of the bioelectrical impedance on the basis of the voltage measured by the voltage measuring section 33 and the reference current. The operating section 38b has the same functions as those of the operating section 38 of the condition information processing apparatus 100A in the first embodiment and the second embodiment in the respect that it executes the above described processing. However, the operating section 38b is different from the operating section 38 of the condition information processing apparatus 100A in the first embodiment and the second embodiment in the respect that the operating section 38b does not execute the measurement of the ratio of the bioelectrical impedance and the measurement of the condition score.

The communication interface 42 of the body composition analyzer 200 is capable of sending and receiving data between the body composition analyzer 200 and the condition information processing apparatus 100B through the communication interface 55 of the condition information processing apparatus 100B. In the embodiment, the measured weight and the measured bioelectrical impedance are sent from the body composition analyzer 200 through the communication interface 42.

Since the function of each section other than the operating section 38b and the communication interface 42 of the body composition analyzer 200 is the same as that of each section of the condition information apparatus 100A described in the first embodiment, the same reference numeral as that used for the each section of the condition information apparatus 100A is used. The explanation of each section other than the operating section 38b and the communication interface 42 of the body composition analyzer 200 will be omitted.

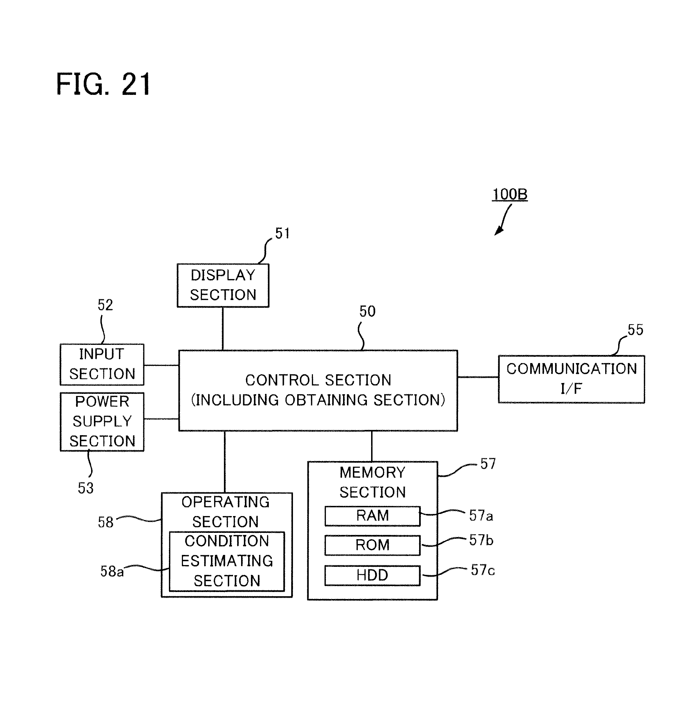

Fig. 21 is a block diagram showing an internal structure of the condition information processing apparatus 100B shown in Fig. 19. Each functional section shown in Fig. 21 is configured of hardware such as a device, equipment, a program for the condition information processing apparatus which is installed and executed, or combination thereof, so that it indicates a functional unit for achieving a predetermined operation.

As shown in Fig. 21, the condition information processing apparatus 100B includes a control section 50, a display section 51, an input section 52, a power supply section 53, a communication interface 55, and a memory section 57, an operating section 58 and a condition estimating section 58a.

The control section 50, which is a processor such as a CPU or a DSP (Digital Signal Processor), is connected to each section electrically to control the operation of each section. In the third embodiment, the control section 31 has functions to calculate the ratio of the bioelectrical impedances on the basis of the bioelectrical impedances received from the body composition analyzer 200, to calculate the condition score on the basis of the calculated ratio of the bioelectrical impedances and the past ratios of the bioelectrical impedances recorded as the measurement history, and to display the result of the estimation of the condition, etc., on the basis of the condition score. In the embodiment, control section 50 has a function of an obtaining section. The obtaining section of the embodiment has a function to obtain the bioelectrical impedances sent from the body composition analyzer 200 through the communication interface 55. The communication interface 55 is used for sending and receiving data between the body composition analyzer 200 and the condition information processing apparatus 100B through the communication network NET.

The display section 51 displays data, etc., sent from the control section 50. In third embodiment, the display section 51 mainly displays transition of the condition score of the user, the estimation of the condition, and advice, etc. A liquid crystal display such as a full-dot LCD can be utilized as the display section 51, for example.

The input section 52 is a section for conducting various types of settings of the condition information processing apparatus 100B. In the embodiment, the input section 52 includes a keyboard used for a personal computer, etc., or a touch panel used for a smart phone, etc.

The power supply section 53 supplies an electrical power to each section of an electric power system of the condition information processing apparatus 100B. In third embodiment, a battery or an external power unit, which supplies electrical power to operate the condition information processing apparatus 100B, can be utilized as the power supply section 53.

The memory section 57 includes a RAM 57a, a ROM 57b, and a HDD (Hard Disc Drive), which stores data of various types of information, programs, and the like. The RAM 57a temporarily stores the obtained bioelectrical impedances and the result of operation, and the like. The ROM 57b stores a program for booting the apparatus, a computing equation of the condition score relating to a predetermined estimation of condition and the like. The HDD 57c stores a program for the condition information processing apparatus. In the HDD 57c, the ratio of the bioelectrical impedances and the condition scores as scores, which are calculated by the operating section 58, are recorded as the measurement history together with additional information associated with each of the condition scores. Since the additional information is the same as that of the first embodiment, the explanation of it will be omitted.

The operating section 58 performs various kinds of arithmetic processing such as the calculation of the ratio of the bioelectrical impedances, the calculation of the condition score. The condition estimating section 58a performs the estimating processing of the condition on the basis of the past condition score recorded as the measurement history.

The operation of the body composition analyzer 200 and the condition information processing apparatus 100B will be described.

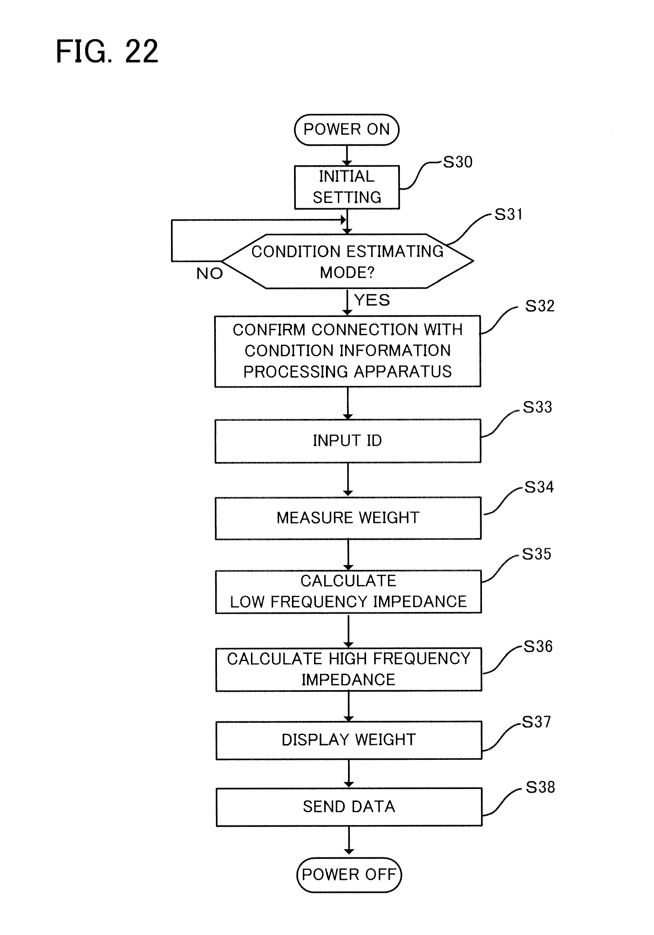

In Fig. 22, a flow chart of the operation of the body composition analyzer 200 in the third embodiment is shown. As shown in Fig. 22, when the input section 22 is operated, the power of the body composition analyzer 200 is turned on, so that an initial setting of the apparatus is performed. Furthermore, a message for requesting the user to select an operation mode is displayed in the display section 21 by the instruction of the control section 31 (Step S30). The user can select the operation mode by using the setting key 22, the up key 22b, and the down key 22c.

The control section 31 of the body composition analyzer 200 determines whether or not an estimating mode of the body condition is selected as the operation mode (Step S31), so that if another mode other than the estimating mode of the body condition is selected (Step S31:NO), it executes the processing of each operation mode. In the flow chart shown in Fig. 22, to simplify the explanation, it is described that the control section 31 is in a wait condition until the estimating mode of the body condition is selected.

When the control section 31 of the body composition analyzer 200 determined that the estimation mode of the body condition is selected (Step S31:YES), the control section 31 confirms the connection between the body composition analyzer 200 and the condition information processing apparatus 100B (Step S32). Specifically, a starting data indicating a start of the processing is sent from the body composition analyzer 200 to the condition information processing apparatus 100B, so that the body composition analyzer 200 receives ready data, which indicates that the preparation for sending data to the body composition analyzer 200 and for receiving data from the body composition analyzer 200 is ready, from the condition information processing apparatus 100B. Then, the body composition analyzer 200 sends confirmation data, which indicates that the ready data can be received, to the condition information processing apparatus 100B. The control section 31 of the body composition analyzer 200 confirms the connection with the condition information processing apparatus 100B when the confirmation data can be received. If the data from the condition information processing apparatus 100B cannot be received, a message indicating the occurrence of an error may be displayed on the display section 21 by the instruction of the control section 31.

When the connection with the condition information processing apparatus 100B is confirmed, a message for requesting the user to input an ID, which identifies the user uniquely, is displayed on the display section 21 by the instruction of the control section 31 of the body composition analyzer 200. The control section 31 temporarily stores the ID input through the input section 22 in the memory section 34 (Step S33). There is a possibility that the body composition analyzer 200 will be used by multiple users. Multiple body composition analyzers 200 can be connected with the condition information processing apparatus 100B. Therefore, the input of the ID is requested in the embodiment.

The user steps on the main unit 2 of the body composition analyzer 200 to apply the load to the main unit 2, the user's weight is calculated on the basis of the load signal output from the weight measuring section 36 by the operating section 38b (Step S34). Under the condition that the body surface of the user is attached to the bioelectrical impedance electrodes 23, 24, the low frequency of 5 kHz is set by the frequency setting section 32c. The alternating current of 5 kHz is impressed to the first current applying electrode 23a and the second current applying electrode 23b through the reference current detecting section 32a by the alternating current applying section 32b. The voltage between the first voltage measuring electrode 24a and the second voltage measuring electrode 24b is measured by the voltage measuring section 33. The measured voltage is converted to digital signals by the A/D converting section 35. The operating section 38b calculates the bioelectric impedance on the basis of the digital signal corresponding to the voltage, and the reference current impressed by the alternating current applying section 32b (Step S35). When the calculation of the bioelectrical impedance at the time of impressing the alternating current of 5 kHz is finished, as in the case of the low frequency, the high frequency of 250 kHz is set by the frequency setting section 32c. The alternating current of 250 kHz is impressed to the first current applying electrode 23a and the second current applying electrode 23b through the reference current detecting section 32a by the alternating current applying section 32b. The voltage between the first voltage measuring electrode 24a and the second voltage measuring electrode 24b is measured by the voltage measuring section 33. The measured voltage is converted to digital signal by the A/D converting section 35. The operating section 38b calculates the bioelectric impedance on the basis of the digital signal corresponding to the voltage, and the reference current impressed by the alternating current applying section 32b (Step S36).

When the calculation of the bioelectrical impedance $Z_5$ at the timing of impressing the alternating current of 5 kHz and the bioelectrical impedance $Z_{250}$ at the timing of impressing the alternating current of 250 kHz is finished, the control section 31 of the body composition analyzer 200 displays the calculated weight on the display section 21 (Step S37). The operating section 38 sends the calculated bioelectrical impedance $Z_5$ at the timing of impressing the alternating current of low frequency of 5 kHz and the calculated impedance $Z_{250}$ at the timing of impressing the alternating current of high frequency of 250 kHz together with the temporarily stored user's ID and weight to the condition information processing apparatus 100B (Step S38).

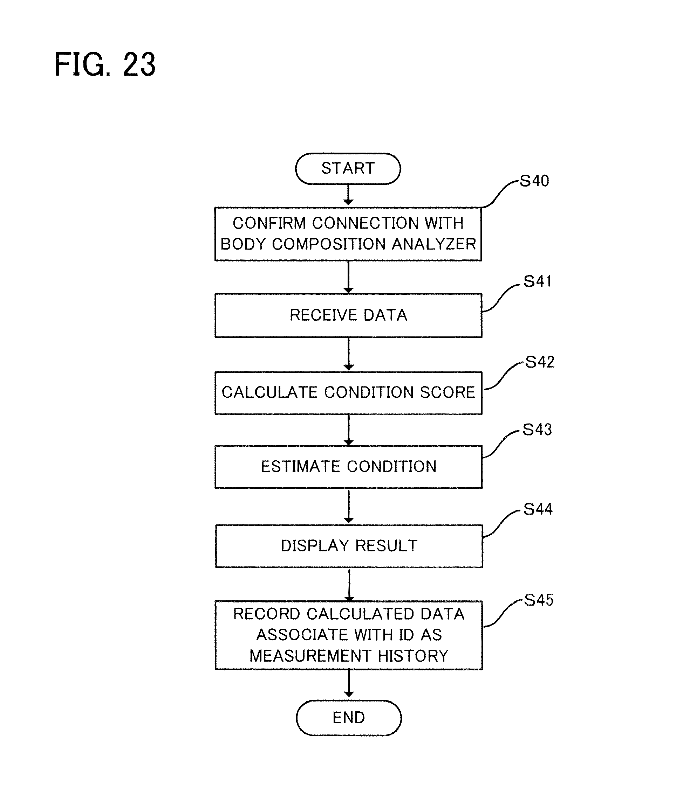

The operation of the condition information processing apparatus 100B will be described by referring to Fig. 23. Fig. 23 is a flow chart showing the operation of the condition information processing apparatus 100B. The flow chart shown in Fig. 23 assumes that a program for estimating condition is activated.

The control section 50 of the condition information processing apparatus 100B confirms the connection between the body composition analyzer 200 and the condition information processing apparatus 100B (Step S40). Specifically, when the control section 50 of the condition information processing apparatus 100B receives starting data indicating the start of processing sent from the body composition analyzer 200, the control section 50 sends ready data , which indicates that the preparation for sending and receiving data is ready, to the body composition analyzer 200. When the control section 50 receives confirmation data, which indicates that the ready data can be received, from the body composition analyzer 200, the control section 50 confirms the connection with the body composition analyzer 200.

When the calculated bioelectrical impedance $Z_5$ at the timing of impressing the alternating current of low frequency of 5 kHz and the calculated impedance $Z_{250}$ at the timing of impressing the alternating current of high frequency of 250 kHz together with the temporarily stored user's ID and weight are sent from the body composition analyzer 200, the control section 50 of the condition information processing apparatus 100B receives the user's ID, weight, and the bioelectrical impedances (Step S41), so that it temporarily stores them in the RAM 57a or the HDD 57c.

The operating section 58 of the condition information processing apparatus 100B calculates the ratio $Z_5/Z_{250}$ of the received bioelectrical impedances to calculate the condition score Ti relating to the body condition on the basis of the calculated ratio $Z_5/Z_{250}$ of the received bioelectrical impedances (Step S42). The calculation of the condition score Ti is executed by using the equation (7) as in the case of the first embodiment. Since the details of the measurement processing of the condition score Ti are the same as those of the first embodiment, explanation thereof will be omitted.

After the condition score Ti is calculated, the condition estimating section 58a of the body composition analyzer 200 estimates the condition of the body on the basis of the calculated condition score and the past condition score recorded as the measurement history (Step S43). Since the details of the estimating processing of the condition are the same as those of the first embodiment, explanation thereof will be omitted.

When the estimation of the body condition is finished, the value of the condition score and the result of the estimation of the condition are displayed on the display section 51 by the instruction of the control section 51 (Step S44). The control section 51 stores the calculated ratio of the bioelectrical impedances and the calculated condition score Ti together with the user's ID and the additional information associated with the condition score Ti in the memory section 57 as the measurement history (Step S45). As is the case of the first embodiment, if the amount of data recorded as the measurement history is small, the above described estimation of the condition is not executed, but only the ratio $Z_5/Z_{250}$ of the received bioelectrical impedances or only a condition score is displayed on the display section 51 by the instruction of the control section 50.

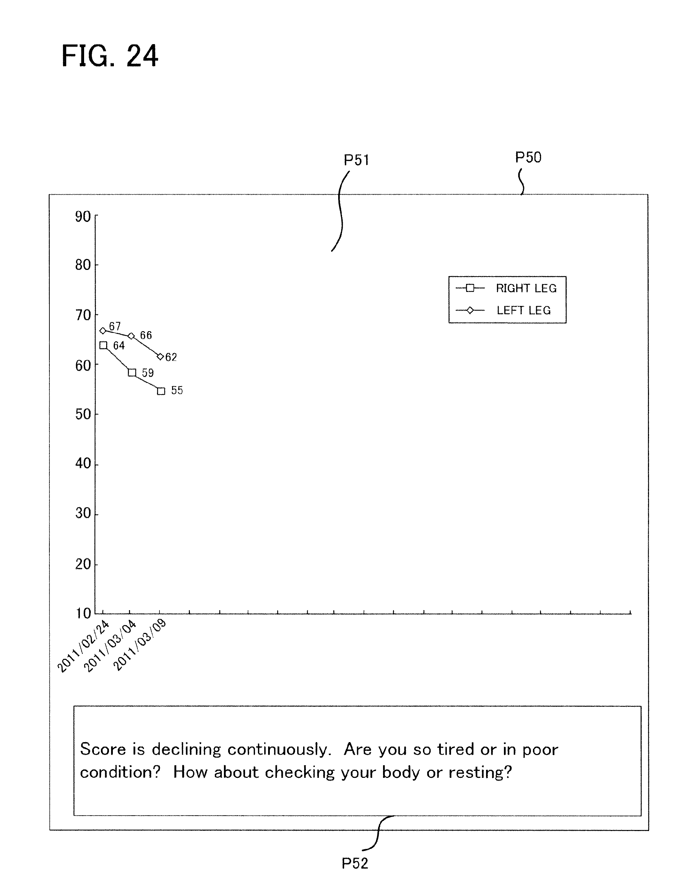

A display example of the estimation of the condition in the condition information processing apparatus 100B is shown in Figs. 24 to 27. Figs. 24 to 27 shows an example of an estimation result displaying page P50 displayed on the display section 51 of the condition information processing apparatus 100B. As shown in Figs. 24 to 27, the estimation result displaying page P50 includes a graph area P51 indicating a graph of the change of the condition score, a message area P52 indicating the change of the condition score, the estimated user's condition, advice and the like. Since a display area in the first embodiment is small, only a message is displayed on the display section 21. However, in the third embodiment, since the display section 50 is a display of a personal computer, a smart phone, or the like, the graph indicating the change of the condition score is displayed on the graph area P51, and the estimated condition and a message such as advice are displayed on the message area P52.

Fig. 24 is a diagram corresponding to Fig. 8 explained in the first embodiment. An example shown in Fig. 24 is one in which the calculated condition score Ti is higher than 50 points, but it continuously declines compared to previous values two or more times. If the newly calculated condition score Ti is higher than 50 points, but it continuously declines compared to previous values two or more times, it is estimated that there is a change of condition, such as inflammation or swelling, of which the user is unaware, and there is a possibility of injury or disease. In third embodiment, the message, "Score is declining continuously. Are you so tired or in poor condition? How about checking your body or resting?" is displayed on the message area P52.

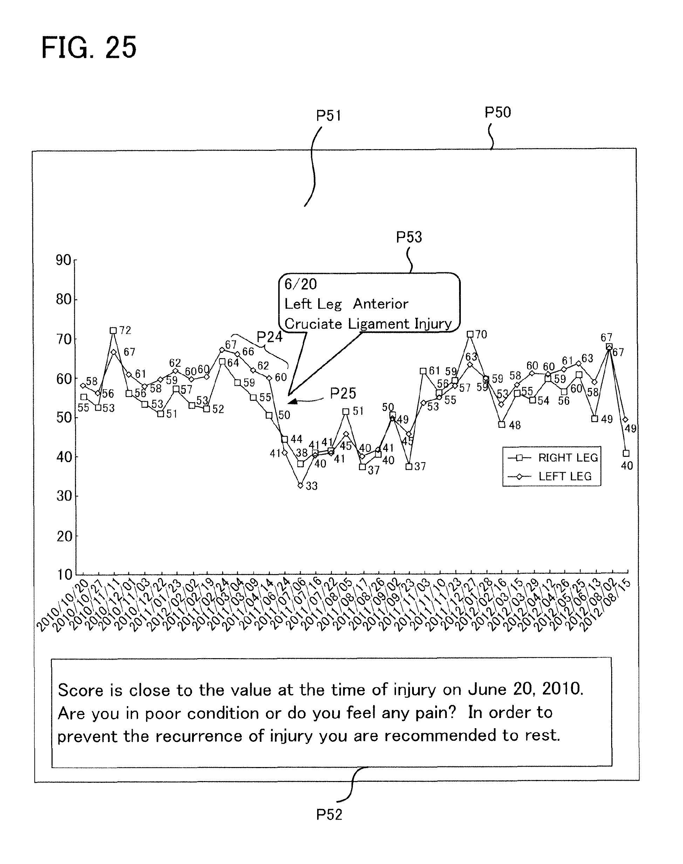

Fig. 25 is a diagram corresponding to Fig. 11 explained in the first embodiment. Fig. 25 is a diagram for explaining an example in which the pattern (P24) of the change of the condition score Ti from February 24, 2011, to April 14, 2011 indicates a similar tendency as the stored pattern (P21, P23 in Fig. 10) of the change of the condition score Ti from February 24, 2010, to April 14, 2010. In this example, the condition score Ti (P25 in Fig. 10) calculated around April 24, 2011, is close to the condition score Ti (P22 in Fig. 10) at the time of the anterior cruciate ligament injury.

In third embodiment, a warning message, "Score is close to the value at the time of injury on June 20, 2010. Are you in poor condition or do you feel any pain? In order to prevent the recurrence of injury, you are recommended to rest." is displayed on the message area P52. In third embodiment, a pop-up message, "6/20 Left Leg Anterior Cruciate Ligament Injury", is displayed. Thus, if the user seeing these messages takes a rest, or decreases the amount of practice, it is possible to prevent of the occurrence of injury or disease.

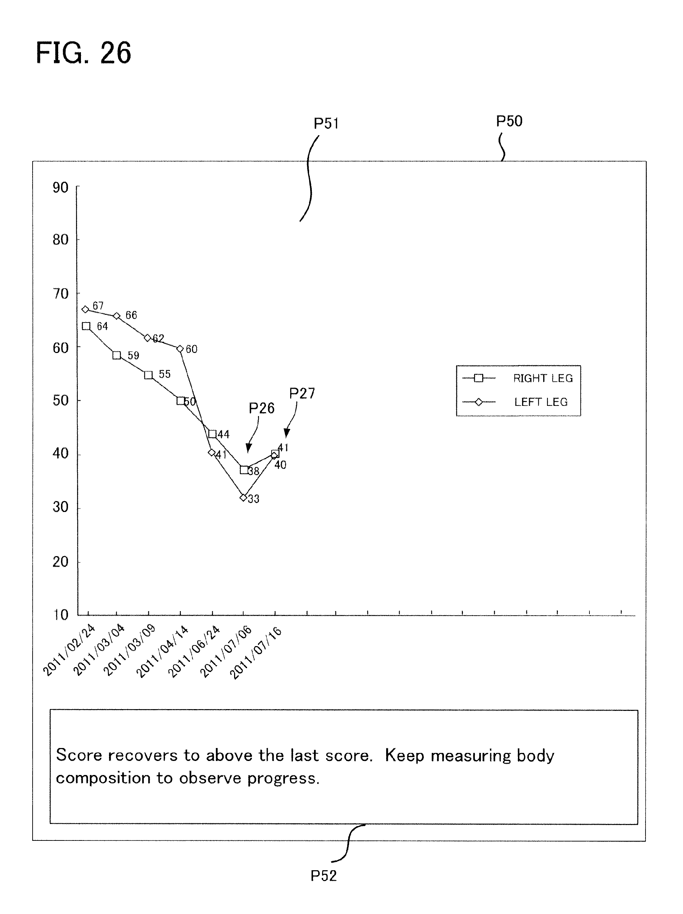

Fig. 26 is a diagram corresponding to Fig. 12 explained in the first embodiment. Fig. 26 is a diagram for explaining an example in which the condition score Ti (P26) calculated on July 6, 2011, falls below 40 points, so that the condition score Ti (P27) calculated on July 16, 2011, exceeds the last score. In this case, the message, "Score recovers to above the last score. Keep measuring body composition to observe progress." is displayed on the message area P52.

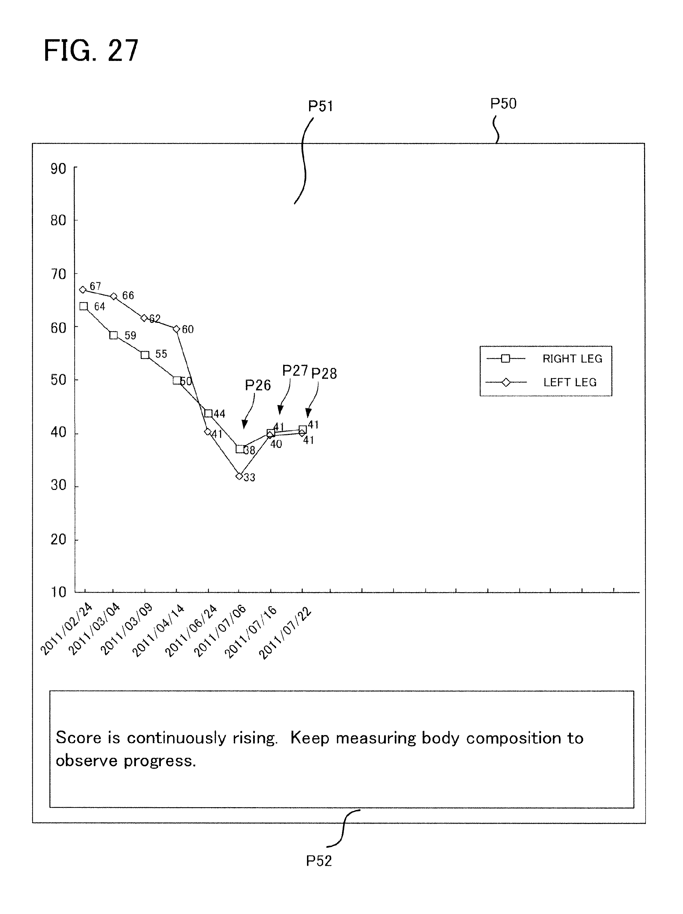

Fig. 27 is a diagram corresponding to Fig. 13 explained in the first embodiment. Fig. 27 is a diagram for explaining an example in which the condition score Ti recovers from the condition shown in Fig. 26, so that the condition score Ti (P28) calculated on July 22, 2011, exceeds the last score, that is, the condition score Ti continuously rises two or more times. In this case, the message, "Score is continuously rising. Keep measuring body composition to observe progress." is displayed on the message area P52.

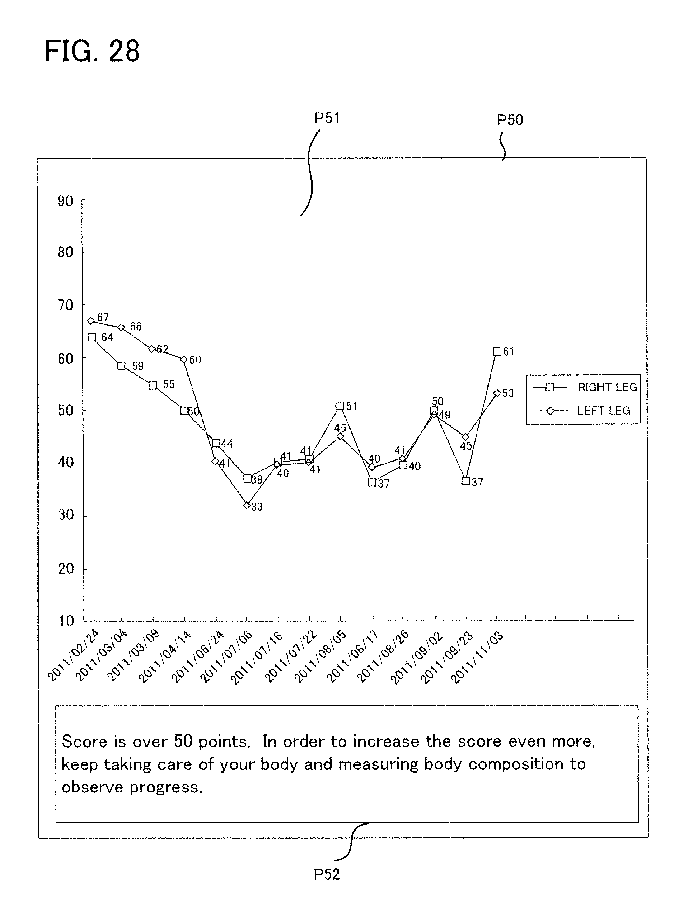

Fig. 28 is a diagram corresponding to Fig. 14 explained in the first embodiment. Fig. 28 is a diagram for explaining an example in which the condition score Ti is recovering from the condition shown in Fig. 27, so that it is over 50 points. In this case, the message, "Score is over 50 points. In order to increase the score even more, keep taking care of your body and measuring body composition to observe progress." is displayed on the message area P52.

As described above, according to the embodiment, the ratio of the bioelectrical impedances and the condition score Ti are calculated on the basis of the bioelectrical impedances, which are measured by the body composition analyzer 200, by the condition information processing apparatus 100B. Furthermore, the condition is estimated. Since not only messages such as the estimated condition and advice, but also the graph indicating the change of the condition score, are displayed, the user can understand the transition of the condition easily and appropriately, so that the user can recover and maintain the user's condition appropriately.

According to the embodiment, since multiple body composition analyzers 200 can be connected to the condition information processing apparatus 100B, if the condition information processing apparatus 100B is embodied by a server connected on the Internet, a personal computer, or the like, it is possible to calculate the ratio of the bioelectrical impedances on the basis of the bioelectrical impedances, which are measured by the body composition analyzers 200 located on the user's home or a training room away from the user's home, and to calculate the condition score on the basis of the ratio of the bioelectrical impedances to estimate the condition.

FOURTH EMBODIMENT

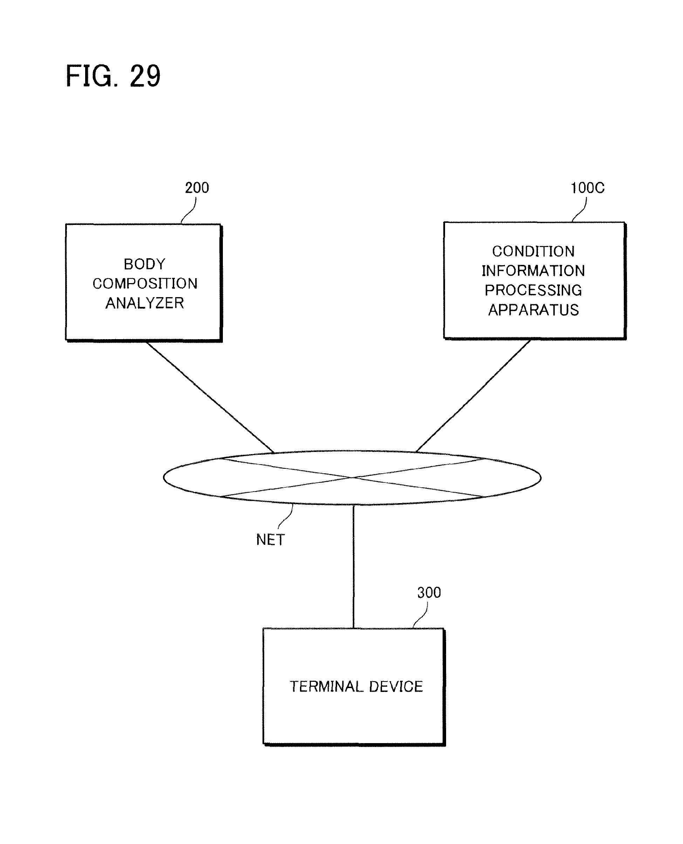

The fourth embodiment of the present invention will be described by referring to Figs. 29 to 34.

In the third embodiment, the measurement of the weight and the measurement of the information relating to the bioelectrical impedance are executed by the body composition analyzer 200. Furthermore, the calculation of the ratio of the bioelectrical impedances, the calculation of the condition score, and the estimation of the condition are executed by the condition information processing apparatus 100B.

The fourth embodiment is common to the third embodiment in the respect that the measurement of the weight and the measurement of the information relating to the bioelectrical impedance are executed by the body composition analyzer 200, and the calculation of the ratio of the bioelectrical impedances, the calculation of the condition score are executed by the condition information processing apparatus 100C. However, the fourth embodiment is different from the third embodiment in the respect that the estimation of the condition is executed by a terminal device 300.

In the fourth embodiment, the alternating current of low frequency of 5 kHz and alternating current of high frequency of 250 kHz are used as the alternating current impressed to the user.

The condition information processing system of the fourth embodiment is shown in Fig. 29. As shown in Fig. 29, the condition information processing system includes the body composition analyzer 200, the condition information processing apparatus 100C, and the terminal device 300. The condition information processing apparatus 100G of the fourth embodiment is an apparatus in which the function to estimate the condition is excluded from the condition information processing apparatus 100B of the third embodiment. The function to estimate the condition is provided in the terminal device 300 of the fourth embodiment. Since the body composition analyzer 200 has the same structure as that of the body composition analyzer 200 explained in the third embodiment, explanation thereof will be omitted in the embodiment.

The condition information processing apparatus 100C is an apparatus such as a personal computer, in which can be installed a program via a CD-ROM or by downloading to execute the program, which can communicate with the body composition analyzer 200 through the communication network NET such as the Internet or a LAN. The condition information processing apparatus 100C may communicate with composition analyzer 200 through a USB (Universal Serial Bus) cable, or Bluetooth, etc., other than the communication network NET. The condition information processing apparatus 100C has functions to calculate the ratio of the bioelectrical impedances on the basis of the bioelectrical impedance received from the body composition analyzer 200, to calculate the condition score on the basis of the ratio of the bioelectrical impedances, by installing a program for the condition information processing apparatus and executing the program.

The terminal device 300 is a device such as a personal computer, or a smart phone, in which can be installed a program through a CD-ROM or by downloading to execute the program, which can communicate with the condition information processing apparatus 100C through the communication network NET such as the Internet or a LAN. The terminal device 300 may communicate with the condition information processing apparatus 100C through a USB (Universal Serial Bus) cable, or Bluetooth, etc., other than the communication network NET. The terminal device 300 has functions to analyze the change of the condition on the basis of the condition score received from the condition information processing apparatus 100C to estimate the condition, and to show the change of an index relating to the condition as the result of the estimation graphically, and to show advice on the condition and the like, by installing a program for the condition information processing apparatus and executing the program.

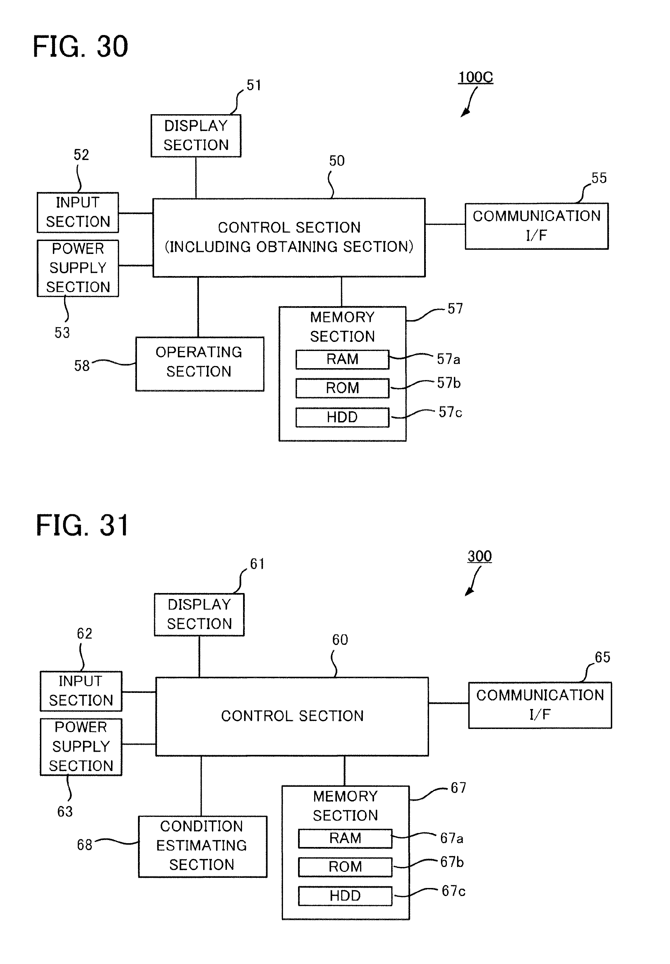

Fig. 30 is a block diagram showing an internal structure of the condition information processing apparatus 100C. The condition information processing apparatus 100C is different from the condition information processing apparatus 100B shown in Fig. 21 in the respect that it does not include a condition estimating section.

The communication interface 55 of the condition information processing apparatus 100C is different from the communication interface 55 of the condition information processing apparatus 100B in the respect that it makes it possible to communicate between the body composition analyzer 200 and the condition information processing apparatus 100C. Furthermore, the communication interface 55 of the condition information processing apparatus 100C is different from the communication interface 55 of the condition information processing apparatus 100B in the respect that it makes it possible to communicate between the terminal device 300 and the condition information processing apparatus 100C.

Since the other structures of the condition information processing apparatus 100C are the same as those of the condition information processing apparatus 100B shown in Fig. 21, explanation thereof will be omitted.

Fig. 31 is a block diagram showing an internal structure of the terminal device 300. As shown in Fig. 31, the terminal device 300 includes a control section 60, a display section 61, an input section 62, a power supply section 63, a communication interface 65, a memory section 67, and a condition estimating section 68.

The control section 60, which is a processor such as a CPU or a DSP (Digital Signal Processor), is connected to each section electrically to control the operation of each section.

The display section 61 displays data, etc., sent from the control section 60. In the fourth embodiment, the display section 61 mainly displays transition of the condition score of the user, the estimation of the condition, and advice, etc. A liquid crystal display such as a full-dot LCD can be utilized as the display section 61, for example.

The input section 62 is a section for conducting various types of settings of the terminal device 300. In the embodiment, the input section 62 includes a keyboard used for a personal computer, etc., or a touch panel used for a smart phone, etc.

The power supply section 63 supplies electrical power to each section of an electric power system of the terminal device 300. In the fourth embodiment, a battery or an external power unit, which supplies electrical power to operate the terminal device 300, can be utilized as the power supply section 63.

The communication interface 65 is used for sending and receiving data between the terminal device 300 and the condition information processing apparatus 100C. In the fourth embodiment, the condition score and the like sent from the condition information processing apparatus 100C is input in the control section 60 through the communication network NET, the communication interface 55, and the communication interface 65.

The memory section 67 includes a RAM 67a, a ROM 67b, and a HDD (Hard Disc Drive) 67c, which stores data of various types of information, programs, and the like. The RAM 67a temporarily stores the obtained bioelectrical impedances and the result of operation, and the like. The ROM 67b stores a program for booting the apparatus. The HDD 67c stores a program for estimating the condition. An SD (Secure Digital) memory and the like may be included in the memory section 67.

The condition estimating section 68 estimates the condition. Specifically, the change of the condition score is analyzed on the basis of the newly calculated condition score Ti and the condition scores Tn recorded as the measurement history to estimate the condition on the basis of the analysis.

The operation of the body composition analyzer 200, the condition information processing apparatus 100C, and the terminal device 300 of the fourth embodiment will be described. Since the operation of the body composition analyzer 200 is the same as that of the body composition analyzer 200 of the third embodiment, the explanation of it will be omitted. In the explanation of the operation of the body composition analyzer 200, the part describing "condition information processing apparatus 100B" is replaced with "condition information processing apparatus 100C".

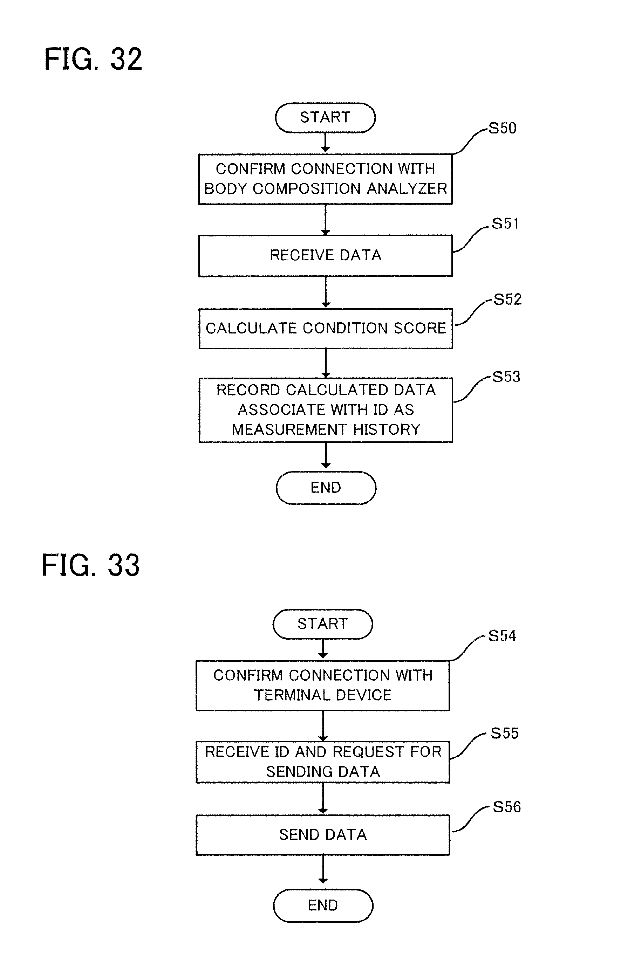

The operation of the condition information processing apparatus 100C will be described by referring to Fig. 32 and Fig. 33. Fig. 32 is a flow chart showing the operation of the condition information processing apparatus 100C in relation to the body composition analyzer 200. Fig. 33 is a flow chart showing the operation of the condition information processing apparatus 100C in relation to the terminal device 300. The flow charts shown in Fig. 32 and Fig. 33 assume that a program for calculating the condition score is activated in the condition information processing apparatus 100C.

The control section 50 of the condition information processing apparatus 100C confirms the connection between the body composition analyzer 200 and the condition information processing apparatus 100C (Step S50). Specifically, when the control section 50 of the condition information processing apparatus 100C receives a starting data indicating a start of the processing sent from the body composition analyzer 200, the control section 50 sends ready data, which indicates that the preparation for sending data to the body composition analyzer 200 and for receiving data from the body composition analyzer 200 is ready, to the body composition analyzer 200. When the control section 50 receives confirmation data, which indicates that the body composition analyzer 200 has received the ready data, from the body composition analyzer 200, so that the control section 50 confirms the connection with the body composition analyzer 200.

When the bioelectrical impedance $Z_5$ at the time of impressing the alternating current of 5 kHz and the bioelectrical impedance $Z_{250}$ at the time of impressing the alternating current of 250 kHz, together with the user's ID and weight are sent from the body composition analyzer 200, the control section 50 of the condition information processing apparatus 100C receives the user's ID, weight, and each bioelectrical impedance (Step S51), so that the control section 50 temporarily stores the user's ID, weight, and each bioelectrical impedance in the RAM57a or the HDD 57c.

The operating section 58 of the condition information processing apparatus 100C calculates the ratio $Z_5/Z_{250}$ of the received bioelectrical impedances to calculate the condition score Ti relating to the body condition on the basis of the calculated ratio $Z_5/Z_{250}$ of the received bioelectrical impedances (Step S52). The calculation of the condition score Ti is executed by using the equation (7) as in the case of the first embodiment. Since the details of the measurement processing of the condition score Ti are the same as those of the first embodiment, explanation thereof will be omitted.

When the calculation of the condition score is finished, the control section 50 records the calculated condition score Ti together with the additional information and the user's ID as the measurement history in HDD57c (Step S53).

The operation of the condition information processing apparatus 100C in relation to the terminal device 300 will be described by referring to Fig. 33. The control section 50 of the condition information processing apparatus 100C confirms the connection between the terminal device 300 and the condition information processing apparatus 100C (Step S54). Specifically, when the control section 50 of the condition information processing apparatus 100C receives a starting data indicating a start of the processing sent from the terminal device 300, the control section 50 sends ready data, which indicates that the preparation for sending data to terminal device 300 and for receiving data from terminal device 300 is ready, to the terminal device 300. When the control section 50 receives confirmation data, which indicates that the terminal device 300 has received the ready data, from the terminal device 300, so that the control section 50 confirms the connection with the terminal device 300.

When the ID for identifying the user is sent from the terminal device 300, the control section 50 of the condition information processing apparatus 100C receives the ID (Step S55) to temporarily store the ID in the RAM 57a or the HDD 57c. The control section 50 sends the data of the condition score associated with the received ID among the condition score recorded in the HDD 57c to the terminal device 300 (Step S56).

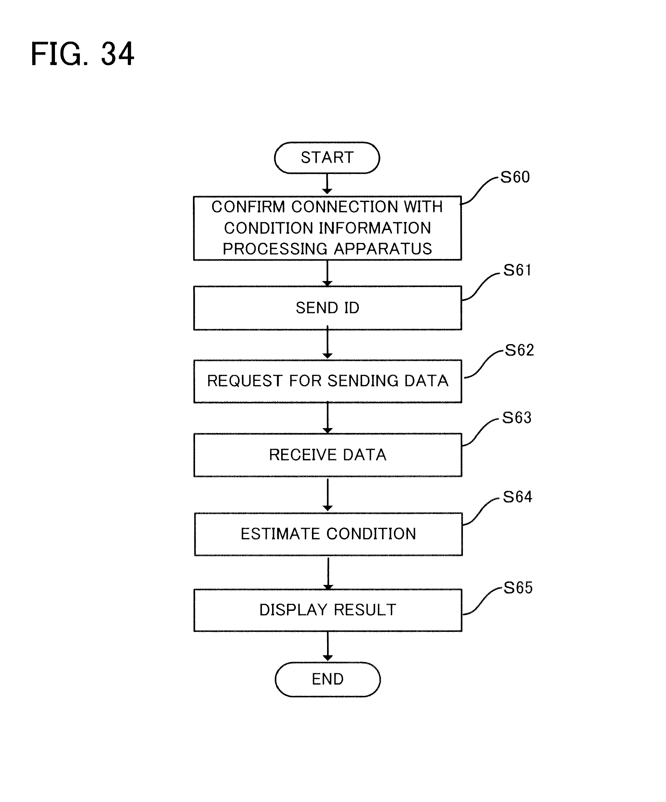

The operation of the terminal device 300 will be described by referring to Fig. 34. Fig. 34 is a flow chart showing the operation of the terminal device 300. The flow chart shown in Fig. 34 assumes that a program for estimating the condition is activated in the terminal device 300.

The control section 60 of the terminal device 300 confirms the connection between the terminal device 300 and the condition information processing device 100C (Step S60). Specifically, the control section 60 of the terminal device 300 sends a starting data indicating a start of the processing sent to the condition information processing device 100C. When the control section 60 receives ready data, which indicates that the preparation for sending data to terminal device 300 and for receiving data from terminal device 300 is ready, from the condition information processing device 100C, the control section 60 sends confirmation data, which indicates that the terminal device 300 has received the ready data, to the condition information processing device 100C, so that the control section 60 confirms the connection with the condition information processing device 100C.

The control section 60 sends the ID, which identifies the user and is input through the input section 62, to the condition information processing apparatus 100C (Step S61). In order to let the user input the ID through the input section 62, the message "Enter Your ID" may be displayed on the display section 61 by the instruction of the control section 60.

After sending the ID, the control section 60 of the terminal device 300 requests the condition information processing apparatus 100C to send the condition score (Step S62). In response to this request, if the condition score is sent from the condition information processing apparatus 100C, the control section 60 of the terminal device 300 receives this condition score to store it in the RAM 67a (Step S63). In the condition score, the newly calculated condition score and the past condition scores recorded as the measurement history are included.

The condition estimating section 68 of the terminal device 300 estimates the condition on the basis of the newly calculated condition score included in the received condition score and the past condition scores recorded as the measurement history (Step S64).

When the estimation of the body condition is finished, the control section 60 displays the value of the condition score and the result of the estimation of the condition on the display section 61 (Step S65). If the amount of data recorded as the measurement history is small, the above-described estimation of the condition is not executed, but only the ratio $Z_5/Z_{250}$ of the bioelectrical impedances or the condition score is displayed on the display section 61 by the instruction of the control section 60.

As for contents displayed on the display section 61, as show in Figs. 24 to 27, the estimated condition and the message such an advice, together with the graph showing the change of the condition score, may be displayed. If the display section 61 is small, only the estimated condition and the message such an advice may be displayed.

As described above, according to the embodiment, the condition score Ti is calculated on the basis of the bioelectrical impedances, which are measured by the body composition analyzer 200, by the condition information processing apparatus 100C such as a personal computer. Furthermore, the estimated condition and the message such as advice are displayed on the display section 61 of the terminal device 300 such as a smart phone. Thus, the user can understand the transition of the condition easily and appropriately, so that the user can recover and maintain the user's condition appropriately.

According to the embodiment, since multiple body composition analyzers 200 and multiple terminal devices 300 can be connected to the condition information processing apparatus 100C, if the condition information processing apparatus 100C is embodied by a server connected on the Internet, a personal computer, or the like, it is possible to calculate the condition score on the basis of the bioelectrical impedances, which are measured by the body composition analyzers 200 located on the user's home or a training room away from the user's home, and to confirm the result of the estimation of the condition at a place away from the user's home or the training room.

FIFTH EMBODIMENT

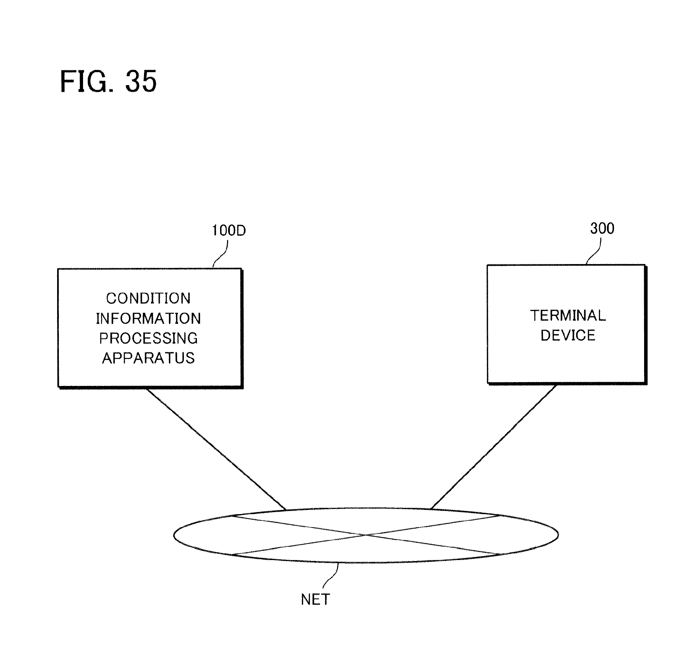

The fifth embodiment of the present invention will be described by referring to Figs. 35 to 37.

In the first embodiment, it is described that one apparatus executes the measurement of the weight, the measurement of the information relating to the bioelectrical impedance, the calculation of the ratio of the bioelectrical impedances, the calculation of the condition score, and the estimation of the condition.

However, the fifth embodiment is different from the first embodiment in the respect that the measurement of the weight, the measurement of the information relating to the bioelectrical impedance, the calculation of the ratio of the bioelectrical impedances, and the calculation of the condition score are executed by the condition information processing apparatus 100D, and the estimation of the condition is executed by the terminal device 300.

In comparison with the fourth embodiment, the function of the body composition analyzer 200 and the function of the condition information processing apparatus 100C in the fourth embodiment are combined into the function of the condition information processing apparatus 100D.

In the fifth embodiment, the alternating current of low frequency of 5 kHz and the alternating current of high frequency of 250 kHz are used as the alternating current impressed to the user.

Fig. 35 is an explanatory diagram showing the structure of the condition processing system in the fifth embodiment. As shown in Fig. 35, the condition information processing system of the fifth embodiment includes the condition information processing apparatus 100D, and the terminal device 300. The condition information processing apparatus 100D of the fifth embodiment is an apparatus in which the function of the condition information processing apparatus 100C is provided with the body composition analyzer 200 in the fourth embodiment. The condition information processing apparatus 100D executes the measurement of the bioelectrical impedance, the calculation of the ratio of the bioelectrical impedances on the basis of the measured bioelectrical impedance, and the calculation of the condition score on the basis of the ratio of the bioelectrical impedances. The terminal device 300 of the fifth embodiment is the same as the terminal device 300 of the fourth embodiment, which has a function to estimate the condition. Since the details of the terminal device 300 are explained in the fourth embodiment, explanation thereof will be omitted in the embodiment.

The condition information processing apparatus 100D has similar appearance to the condition information processing apparatus 100A in the first embodiment, and has functions of the body composition analyzer 200 of the fourth embodiment and the function of the condition information processing apparatus 100C of the fourth embodiment. The condition information processing apparatus 100D is capable of communicating with the terminal device 300 through a communication network NET such as the Internet or a LAN. The condition information processing apparatus 100D may communicate with the terminal device 300 through a USB (Universal Serial Bus) cable, or Bluetooth, etc., other than the communication network NET.

The terminal device 300 is the same as the terminal device 300 of the fourth embodiment, which is a device such as a personal computer, or a smart phone, in which can be installed a program through a CD-ROM or by downloading to execute the program. The terminal device 300 has functions to analyze the change of the condition on the basis of the condition score received from the condition information processing apparatus 100D and the condition scores recorded as the measurement history to estimate the condition, and to show the change of an index relating to the condition as the result of the estimation graphically, and to show advice on the condition and the like.

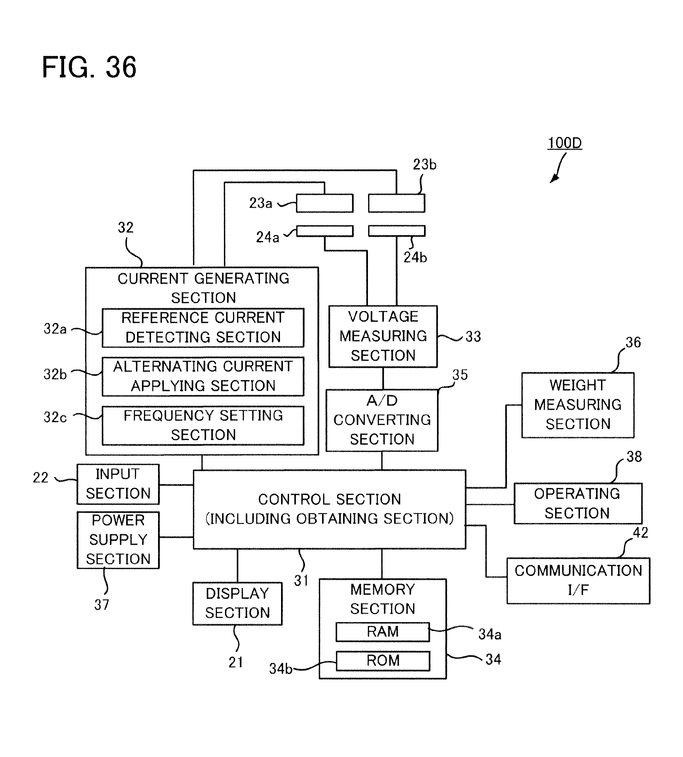

Fig. 36 is a block diagram showing an internal structure of the condition information processing apparatus 100D. The condition information processing apparatus 100D is different from the condition information processing apparatus 100A shown in Fig. 2 in the respect that it does not include a condition estimating section. The condition information processing apparatus 100D is different from the condition information processing apparatus 100A shown in Fig. 2 in the respect that it includes a communication interface 42.

The operating section 38 of the condition information processing apparatus 100D executes the calculation processing of the weight on the basis of the load signal, the calculation processing of the bioelectrical impedance on the basis of the measured voltage, the calculation processing of the ratio $Z_5/Z_{250}$ of the bioelectrical impedance $Z_5$ at the time of impressing the alternating current of the frequency of 5 kHz and the bioelectrical impedance $Z_{250}$ at the time of impressing the alternating current of the frequency of 250 kHz, the calculation processing of the condition score on the basis of the newly calculated ratio of the bioelectrical impedances and the past ratios of the bioelectrical impedances recorded as the measurement history, and the like.

The communication interface 42 of the condition information processing apparatus 100D makes it possible to communicate between the condition information processing apparatus 100D and the terminal device 300, and is used for sending and receiving data between the condition information processing apparatus 100D and the terminal device 300. Since the other structures of the condition information processing apparatus 100D are the same as those of the condition information processing apparatus 100A shown in Fig. 2, explanation thereof will be omitted.

Since the structure of the terminal device 300 is the same as that of the terminal device 300 of the fourth embodiment shown in Fig. 31, explanation of details thereof will be omitted. The condition estimating selection 68 included in the terminal device 300 analyzes the change of the condition score to estimate the condition on the basis of the analysis even in the fifth embodiment. Specifically, the condition estimating selection 68 analyzes the tendency of the change of the condition score and the like on the basis of the newly calculated condition score Ti and the condition scores Tn recorded as the measurement history to estimate the condition on the basis of the analysis.

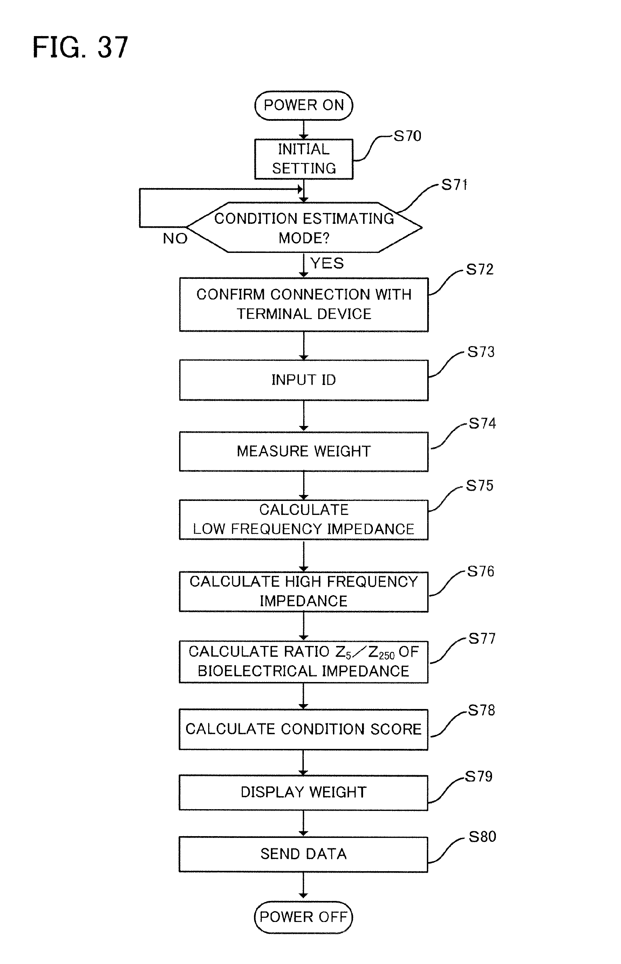

The operation of the condition information processing apparatus 100D will be described by referring to Fig. 37. As shown in Fig. 37, when the input section 22 is operated, the power of the condition information processing apparatus 100D is turned on. Then, an initial setting of the apparatus is performed. Furthermore, a message for requesting the user to select an operation mode is displayed in the display section 21 by the instruction of the control section 31 (Step S70). The user can select the operation mode by using the setting key 22a, the up key 22b, and the down key 22c.

The control section 31 of the condition information processing apparatus 100D determines whether or not an estimating mode of the body condition is selected as the operation mode (Step S71), so that if another mode other than the estimation mode of the body condition is selected (Step S71:NO), it executes the processing of each operation mode. In the flow chart shown in Fig. 37, to simplify the explanation, it is described that the control section 31 is in a wait condition until the estimation mode of the body condition is selected.

When the control section 31 of the condition information processing apparatus 100D determines that the estimating mode of the body condition is selected (Step S71:YES), the control section 31 confirms the connection between the condition information processing apparatus 100D and the terminal device 300 (Step S72). Specifically, the control section 31 of the condition information processing apparatus 100D sends starting data indicating a start of the processing to the terminal device 300. When the control section 31 receives ready data, which indicates that the preparation for sending data to the condition information processing apparatus 100D and for receiving data from the condition information processing apparatus 100D is ready, from the terminal device 300, the control section 31 sends confirmation data, which indicates that the condition information processing apparatus 100D has received the ready data, to the terminal device 300, so that the control section 31 confirms the connection with the terminal device 300.

When the connection with the terminal device 300 is confirmed, a message for requesting the user to input an ID, which identifies the user uniquely, is displayed on the display section 21 by the instruction of the control section 31 of the condition information processing apparatus 100D. The control section 31 temporarily stores the ID input through the input section 22 in the memory section 34 (Step S73). There is a possibility that the condition information processing apparatus 100D is used by multiple users. Multiple condition information processing apparatuses 100D can be connected with the terminal device 300. Therefore, the input of the ID is requested in the embodiment.

The user steps on the main unit 2 of the condition information processing apparatuses 100D to apply the load to the main unit 2, the user's weight is calculated on the basis of the load signal output from the weight measuring section 36 by the operating section 38 (Step S74). Under the condition that the body surface of the user is attached to the bioelectrical impedance electrodes 23 and 24, the low frequency of 5 kHz is set by the frequency setting section 32c. The alternating current of 5 kHz is impressed to the first current applying electrode 23 a and the second current applying electrode 23b through the reference current detecting section 32a by the alternating current applying section 32b. The voltage between the first voltage measuring electrode 24a and the second voltage measuring electrode 24b is measured by the voltage measuring section 33. The measured voltage is converted to digital signal by the A/D converting section 35. The operating section 38 calculates the bioelectric impedance on the basis of the digital signal corresponding to the voltage and the reference current impressed by the alternating current applying section 32b (Step S75). When the calculation of the bioelectrical impedance at the time of impressing the alternating current of 5 kHz is finished, as in the case of the low frequency, the high frequency of 250 kHz is set by the frequency setting section 32c. The alternating current of 250 kHz is impressed to the first current applying electrode 23a and the second current applying electrode 23b through the reference current detecting section 32a by the alternating current applying section 32b. The voltage between the first voltage measuring electrode 24a and the second voltage measuring electrode 24b is measured by the voltage measuring section 33. The measured voltage is converted to digital signal by the A/D converting section 35. The operating section 38 calculates the bioelectric impedance on the basis of the digital signal corresponding to the voltage and the reference current impressed by the alternating current applying section 32b (Step S76).

When the calculation of the bioelectrical impedance $Z_5$ at the time of impressing the alternating current of 5 kHz and the bioelectrical impedance $Z_{250}$ at the time of impressing the alternating current of 250 kHz is finished, the operating section 38 of the condition information processing apparatuses 100D calculates the ratio $Z_5/Z_{250}$ of the calculated bioelectrical impedance $Z_5$ and the calculated bioelectrical impedance $Z_{250}$ (Step S77). The operating section 38 calculates the condition score Ti relating to the body condition on the basis of the calculated ratio $Z_5/Z_{250}$ of the bioelectrical impedances and the past ratios $Z_5/Z_{250}$ of the bioelectrical impedances recorded as the measurement history by using the equation (7) (Step S78). As in the case of the second embodiment, if the amount of the data recorded as the measurement history is small, the above described calculation of the condition score is not executed.

When the calculation of the condition score is finished, the control section 31 of the condition information processing apparatus 100D displays the calculated weight on the display section 21 (Step S79) to send the calculated weight, the calculated condition score, and the past condition scores recorded as the measurement history, together with the user's ID, which is temporarily stored, to the terminal device 300 (Step S80).

When the sent weight, newly calculated condition score, past condition scores recorded as the measurement history, and user's ID are received by the terminal device 300, the condition estimating section 68 of the terminal device 300 analyzes the tendency of the change of the body condition on the basis of the newly calculated condition score, and the past condition score recorded as the measurement history to estimate the condition. The control section 60 of the terminal device 300 displays the value of the condition score and the result of the estimation of the condition on the display section 61. Since the details of the operation of the terminal device 300 are the same as those of the fourth embodiment shown in Fig. 34, explanation thereof will be omitted.

As described above, according to the embodiment, the calculation of the weight, the calculation of the bioelectrical impedance, the calculation of the ratio of the bioelectrical impedance, and the calculation of the condition score Ti are executed by the condition information processing apparatus 100D. Then, the estimated condition and the message such as advice are displayed on the display section 61 of the terminal device 300 such as a personal computer. Therefore, the user can understand the transition of the condition easily and appropriately, so that the user can recover and maintain the user's condition appropriately.

According to the embodiment, since the condition information processing apparatus 100D is provided separately from the terminal device 300, it is possible to calculate the condition score on the basis of the bioelectrical impedances, which are measured by the condition information processing apparatus 100D located on the user's home or a training room away from the user's home, and to confirm the result of the estimation of the condition at a place away from the user's home or the training room.

VARIATION 1

In the above-described first embodiment, third embodiment, fourth embodiment, and fifth embodiment, the alternating current of high frequency of 250 kHz and the alternating current of low frequency of 5 kHz are used, so that the ratio $Z_5/Z_{250}$ of the bioelectrical impedance $Z_5$ at the time of impressing the alternating current of low frequency of 5 kHz to the bioelectrical impedance $Z_{250}$ at the time of impressing the alternating current of high frequency of 250 kHz is calculated. Furthermore, the condition score, which is an index relating to the body condition, is calculated on the basis of the calculated ratio $Z_5/Z_{250}$ of the bioelectrical impedances. However, the present invention is not limited to this kind of example. The ratio $Z_{250}/Z_5$ of the bioelectrical impedance $Z_{250}$ at the time of impressing the alternating current of high frequency of 250 kHz to the bioelectrical impedance $Z_5$ at the time of impressing the alternating current of low frequency of 5 kHz may be calculated. Then, the condition score, which is an index relating to the body condition, may be calculated on the basis of the calculated ratio $Z_{250}/Z_5$ of the bioelectrical impedances.

To calculate the condition score, in the variation 1, condition scores, which satisfy a predetermined condition, in the measurement history are aggregated, so that a deviation value of the most recently calculated ratio $Z_{250}/Z_5$ of the

bioelectrical impedances is calculated on the basis of the aggregated condition scores. The equation for calculating this condition score Ti is represented as follows.

$$Ti = 100 - [((Xi - \mu x)/\sigma x) \cdot 10 + 50] \qquad ----- (9)$$

where Xi is a calculated value, $\mu x$ is an average value, $\sigma x$ is a standard deviation value.

The calculated value Xi is newly calculated ratio $Z_{250}/Z_5$ of the bioelectrical impedance. The average value $\mu x$ is the average of multiple ratios $Z_{250}/Z_5$ of the bioelectrical impedances, which are measured in the past. The standard deviation value $\sigma x$ is a value indicating dispersion of the multiple ratios $Z_{250}/Z_5$ of the bioelectrical impedances.

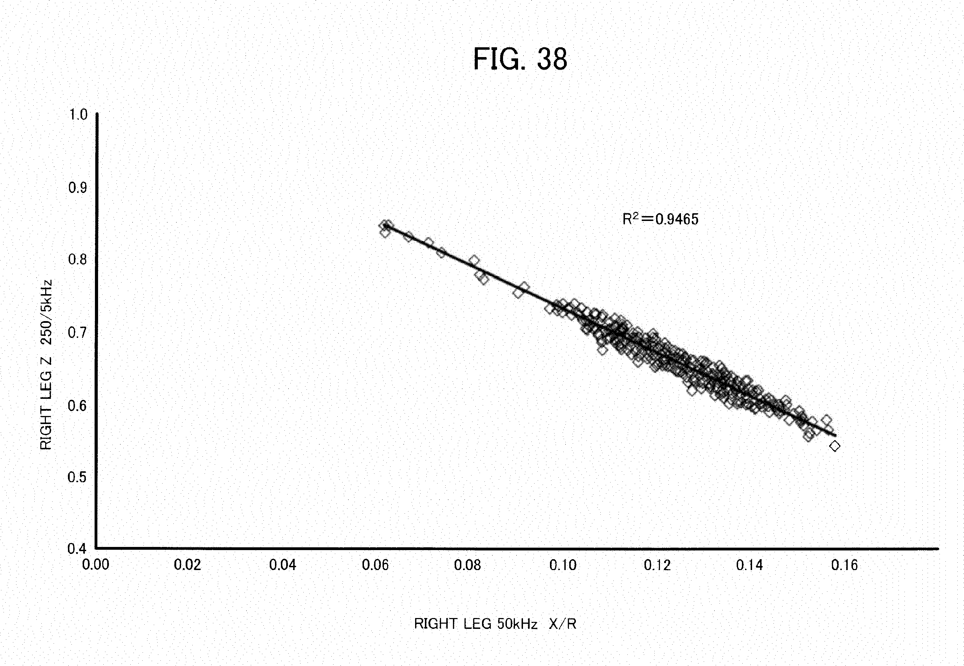

As shown in Fig. 38, even in the ratio $Z_{250}/Z_5$ of the bioelectrical impedances, there is a correlation between the ratio $Z_{250}/Z_5$ of the bioelectrical impedances and the ratio X/R of a reactance component X and a resistance component R in the bioelectrical impedance at the time of impressing the alternating current of the single frequency of 50 kHz. The correlation between the ratio $Z_5/Z_{250}$ of the bioelectrical impedances and the ratio X/R of a reactance component X and a resistance component R in the bioelectrical impedance at the time of impressing the alternating current of the single frequency of 50 kHz is a positive correlation as shown in Fig. 17. The correlation between the ratio $Z_{250}/Z_5$ of the bioelectrical impedances and the ratio X/R of a reactance component X and a resistance component R in the bioelectrical impedance at the time of impressing the alternating current of the single frequency of 50 kHz is a negative correlation as shown in Fig. 38. Therefore, equation (9) for calculating the condition score Ti at the time of using the ratio $Z_{250}/Z_5$ of the bioelectrical impedances is different from the equation (8) for calculating the condition score Ti at the time of using the ratio $Z_5/Z_{250}$ of the bioelectrical impedances.

In the present invention, since the ratio $Z_{250}/Z_5$ of the bioelectrical impedances can be used instead of the ratio $Z_5/Z_{250}$ of the bioelectrical impedances, the ratio $Z_{250}/Z_5$ of the bioelectrical impedances may be calculated by using the equation (9) to calculate the condition score in the first embodiment, the third embodiment, the fourth embodiment, and the fifth embodiment.

VARIATION 2

In the second embodiment, the deviation of the ratio X/R of the reactance component X to the resistance component R is calculated, so that the value of the deviation is used as the condition score Ti. However, the present invention is not limited to this kind of example.

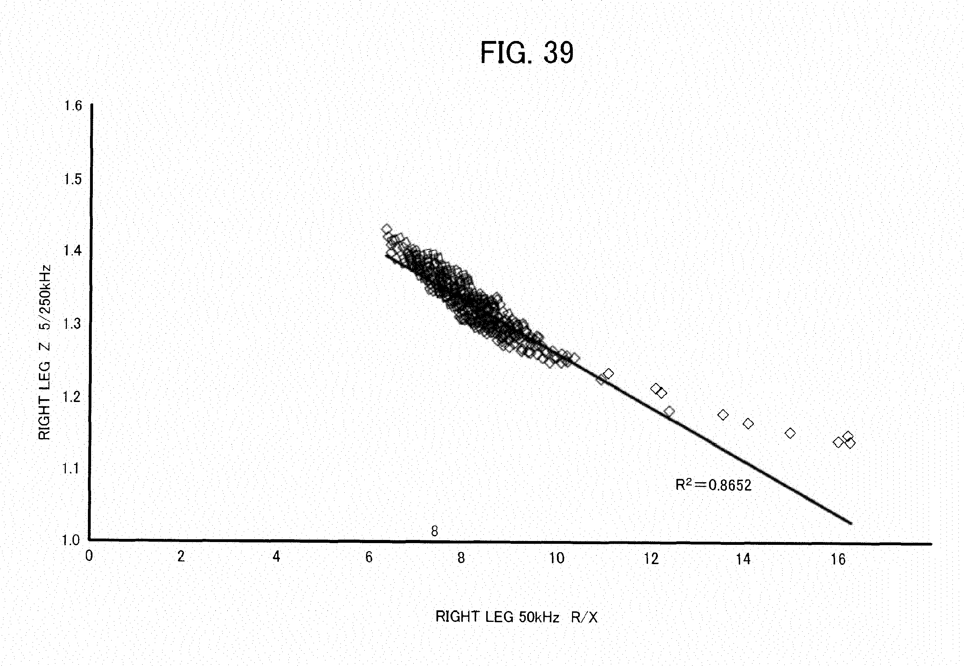

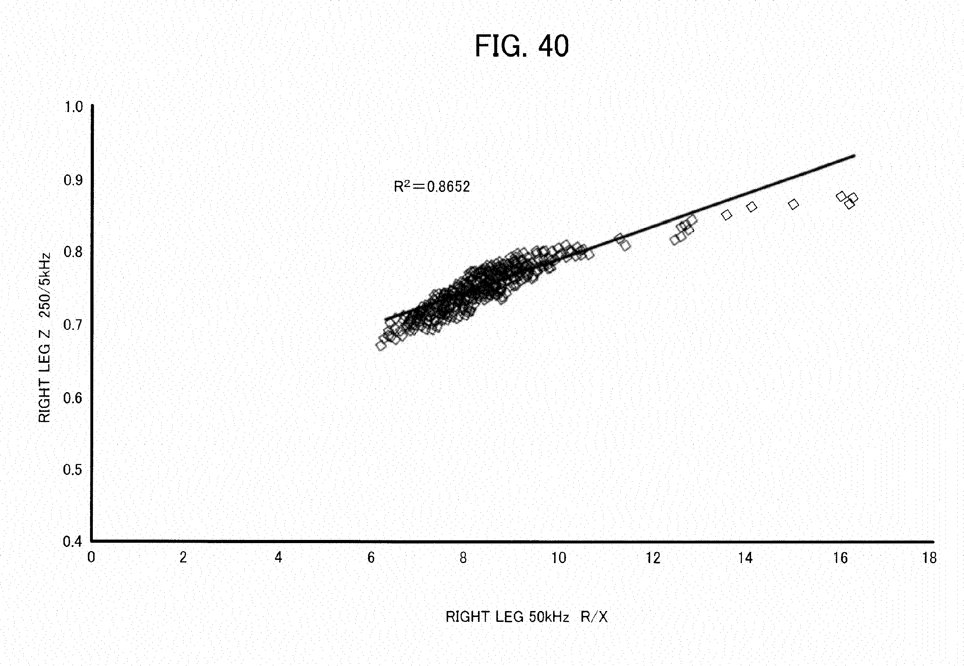

As shown in Fig. 39 and Fig. 40, there is also a correlation between the ratio $Z_5/Z_{250}$ of the bioelectrical impedances and the ratio R/X of a resistance component R to a reactance component X in the bioelectrical impedance. Furthermore, there is a correlation between the ratio $Z_{250}/2_5$ of the bioelectrical impedances and the ratio R/X of a resistance component R to a reactance component X in the bioelectrical impedance. Therefore, the deviation of the ratio R/X of the resistance component R to the reactance component X may be calculated, so that the value of the deviation may be used as the condition score Ti.

In this case, the equation for calculating the condition score Ti can be described as follows:

$$Ti = 100 - [((Xi - \mu x)/\sigma x) \cdot 10 + 50] \qquad ----- (10)$$

where Xi is a calculated value, $\mu x$ is an average value, $\sigma x$ is a standard deviation value.

The calculated value Xi is newly calculated ratio R/X of the resistance component R to the reactance component X. The average value $\mu x$ is the average of multiple ratios R/X of the bioelectrical impedances, which are measured in the past and recorded as the measurement history. The standard deviation value $\sigma x$ is a value indicating dispersion of the multiple ratios R/X of the resistance component R to the reactance component X.

VARIATION 3

In the third embodiment, the fourth embodiment, and the fifth embodiment, the alternating current of low frequency of 5 kHz and the alternating current of high frequency of 250 kHz are impressed to the user. However, the present invention is not limited to this kind of example.

Even in the third embodiment, the fourth embodiment, and the fifth embodiment, as in the case of the second embodiment, the alternating current of a single frequency may be impressed to the user, so that a reactance component X and a resistance component R in a bioelectrical impedance at the time of impressing the alternating current may be calculated to calculate the ratio X/R of the reactance component X to the resistance component R. Thus, the condition score may calculated on the basis of the calculated ratio X/R and the past ratio X/R recorded as the measurement history.

In the third embodiment, the fourth embodiment, and the fifth embodiment, as in the case of the variation 2, the resistance component R and the reactance component X in the bioelectrical impedance may be calculated, so that the ratio R/X of the resistance component R to the reactance component X. Thus, the condition score may calculated on the basis of the calculated ratio R/X and the past ratio R/X recorded as the measurement history.

VARIATION 4

In the body composition analyzer 200 of the third embodiment and the fourth embodiment, and the condition information processing apparatus 100D of the fifth embodiment, an example, in which the weight is displayed after measuring the weight, is explained. Multiple display modes may be provided so that the user can select either one display mode, in which only the weight is displayed, or another display mode, in which the condition score is displayed together with the weight. In this case, the condition information processing apparatus 100B or the condition information processing apparatus 100C may send the condition score calculated therein to the body composition analyzer 200, so that the body composition analyzer 200 may display the condition score. In the condition information processing apparatus 100D of the fifth embodiment, the condition information processing apparatus 100D may display the condition score calculated therein.

VARIATION 5

The ratio $Z_5/Z_{250}$ of the bioelectrical impedances, the ratio $Z_{250}/Z_5$ of the bioelectrical impedances, the ratio X/R of the reactance component X to the resistance component R, and the ratio R/X of the resistance component R to the reactance component X are relating to the change of muscle mass. The change of muscle mass might be measured instead of the change of the condition in the case of calculating the condition score over a long period of time. Therefore, it is preferable to calculate the condition score during a certain period of time, so that the measurement history is reset every certain period of time. For example, in each example described in the specification, such as each embodiment or each variation, the measurement history may be reset every one month.

VARIATION 6

In each example described in the specification, such as each embodiment or each variation, a program for the condition information processing apparatus may be provided as one recorded in a recording medium such as a CD-ROM. Then, the condition information processing apparatus may read the program from the CD-ROM. Furthermore, the condition information processing apparatus may download the program through the Internet.

The memory section in each example described in the specification, such as each embodiment or each variation may include an SD memory card (Secure Digital memory card) and the like in addition to the RAM, ROM, and HDD. A server, which can be accessed through a network, may be utilized as the memory section.

VARIATION 7

In the example, in which a voltage is measured by bringing the user's sole in contact with the electrodes to calculate the bioelectrical impedance on the basis of the voltage, so that the condition is estimated on the basis of the bioelectrical impedance, among the above-described embodiments and variations, it is possible to utilize a holding unit, which holds the user's legs or arms instead of the electrodes contacted with the user's sole. The holding unit may be connected with the main unit 2, so that the control section 31 in the main unit 2 may measure the voltage of the holding unit. Then, the bioelectrical impedance, the ratio $Z_5/Z_{250}$ of the bioelectrical impedances, or the ratio $Z_{250}/Z_5$ of the bioelectrical impedances may be calculated by the operating section 38.

In the example, in which a voltage is measured by bringing the user's sole in contact with the electrodes to calculate the ratio X/R or R/X of a reactance component X and a resistance component R in the bioelectrical impedance, so that the condition is estimated on the basis of the ratio X/R or R/X, among the above described embodiments and variations, it is possible to utilize a holding unit, which holds the user's legs or arms. The holding unit may be connected with the main unit 2, so that the control section 31 in the main unit 2 may measure the voltage of the holding unit. Then, the reactance component X and the resistance component R may be calculated by the operating section 38, so that the ratio X/R or R/X of the reactance component X and the resistance component R may be calculated.

In the example of calculating the ratio of the bioelectrical impedances in each embodiment and each variation, the alternating current of high frequency and the alternating current of low frequency may be impressed to the first current applying electrode 23a, the second current applying electrode 23b, a third current applying electrode and a fourth current applying electrode, which are provided in the holding unit. Then, the bioelectrical impedance may be calculated, so that the ratio $Z_5/Z_{250}$ of the bioelectrical impedances, or the ratio $Z_{250}/Z_5$ of the bioelectrical impedances may be calculated.

In the example of calculating the ratio X/R or R/X of the reactance component X and the resistance component R in the bioelectrical impedances in each embodiment and each variation, the alternating current of the single frequency may be impressed to the first current applying electrode 23a, the second current applying electrode 23b, a third current applying electrode and a fourth current applying electrode, which are provided in the holding unit. Then, the bioelectrical impedance may be calculated, so that the ratio X/R or R/X of the reactance component X and the resistance component R may be calculated.

In each embodiment and each variation, when the condition-estimating mode is selected, the estimation of the condition is executed. The present invention is not limited to this kind of example. When measuring the body composition, body fat, subcutaneous fat and the like by using the apparatus of the present invention, the alternating current of high frequency and the alternating current of low frequency may be impressed at the same time. Then, the bioelectrical impedance may be calculated by measuring each voltage corresponding to each of the alternating currents to calculate the ratio $Z_5/Z_{250}$ of the bioelectrical impedances, or the ratio $Z_{250}/Z_5$ of the bioelectrical impedances. The estimation of the condition may be displayed in addition to the displays of the measured values of the body composition, body fat, subcutaneous fat and the like.

When measuring the body composition, body fat, subcutaneous fat and the like by using the apparatus of the present invention, the alternating current of a single frequency may be impressed at the same time. Then, the voltage may be calculated, so that the ratio X/R or R/X of the reactance component X and the resistance component R in the bioelectrical impedance may be calculated. The estimation of the condition may be displayed in addition to the displays of the measured values of the body composition, the body fat, and subcutaneous fat.

In each embodiment and each variation, the present invention is applied to a type of apparatus, in which the user steps on the surface of the main unit 2. The present invention is not limited to this kind of example. For instance, the present invention may be applied to an apparatus, in which the bioelectrical impedance or the ratio of the reactance component X and the resistance component R in the bioelectrical impedance are calculated to perform various types of measurements, such as an apparatus, in which subcutaneous fat is in the front part of the abdomen.

In each embodiment and each variation, the alternating current of 5 kHz as a low frequency and the alternating current of 250 kHz as a high frequency are used. The present invention is not limited to this kind of example. The value of the frequency of the alternating current may be changed appropriately. For example, as the alternating current of low frequency, the alternating current of 1 to 10 kHz may be used. As the alternating current of high frequency, the alternating current of 200 to 1000 kHz may be used. In each embodiment and each variation, the alternating current of one kind of frequency as the low frequency is used. Furthermore, the alternating current of one kind of frequency as the high frequency is used. The alternating current of various kinds of frequencies may be used. The alternating current of multiple kinds of frequencies may be used for each of the alternating current of low frequency and the alternating current of high frequency. Then, when calculating the ratio $Z_5/Z_{250}$ of the bioelectrical impedances, or the ratio $Z_{250}/Z_5$ of the bioelectrical impedances, the bioelectrical impedance at the time of using the alternating current of a preferable frequency may be used.

In each embodiment and each variation, the alternating current of 50 kHz is used as the alternating current of the single frequency. In the present invention, the value of the frequency is not limited to this kind of example. The value of the frequency may be changed appropriately. For example, the alternating current of any other single frequency having a correlation with the ratio of the bioelectrical impedances at the time of impressing the alternating current of low frequency of 1 to 10 kHz and at the time of impressing the alternating current of high frequency of 200 to 1000 kHz may be used.

In each embodiment and each variation, the alternating current of one kind of frequency as the single frequency is used. The alternating current of various kinds of the single frequencies may be used. The alternating current of multiple kinds of the single frequencies may be used. Then, when calculating the ratio X/R or R/X of the reactance component X and the resistance component R in the bioelectrical impedances, the ratio X/R or R/X of the reactance component X and the resistance component R at the time of using the alternating current of a preferable single frequency may be used.

VARIATION 8

In the example of calculating the bioelectrical impedance in each embodiment and each variation, when calculating the condition score Ti, the average value $\mu x$ and the standard deviation value $\sigma x$ are calculated by using multiple ratios $Z_5/Z_{250}$ of the bioelectrical impedances or multiple ratios $Z_{250}/Z_5$ of the bioelectrical impedances, which are previously measured. The present invention is not limited to this kind of example. For example, the average value $\mu x$ and the standard deviation value $\sigma x$ may be calculated by using the newly calculated ratio $Z_5/Z_{250}$ of the bioelectrical impedance or the newly calculated ratio $Z_{250}/Z_5$ of the bioelectrical impedance in addition to the multiple ratios previously measured. In this case, without aggregating predetermined multiple ratios $Z_5/Z_{250}$ of the bioelectrical impedance or predetermined multiple ratios $Z_{250}/Z_5$ of the bioelectrical impedance, multiple ratios $Z_5/Z_{250}$ of the bioelectrical impedance

or multiple ratios $Z_{250}/Z_5$ of the bioelectrical impedance, which are close to each other in terms of a period of the calculation, may be appropriately selected. In this case, it is possible to estimate the condition in accordance with the user's condition, which changes day by day.

In the example of calculating the ratio X/R or R/X of the reactance component X and the resistance component R in the bioelectrical impedances in each embodiment and each variation, when calculating the condition Ti, the average value $\mu x$ and the standard deviation value $\sigma x$ are calculated by using multiple ratios X/R or R/X , which are previously measured. The present invention is not limited to this kind of example. For example, the average value $\mu x$ and the standard deviation value $\sigma x$ may be calculated by using the newly calculated ratio X/R or R/X in addition to the multiple ratios previously measured. In this case, without aggregating predetermined multiple ratios X/R or R/X, multiple ratios X/R or R/X, which are close to each other in terms of a period of the calculation, may be appropriately selected. In this case, it is possible to estimate the condition in accordance with the user's condition, which changes day by day.

VARIATION 9

In each embodiment and each variation, when calculating the condition score Ti, the average value $\mu x$ and the standard deviation value $\sigma x$ are calculated by using the ratio $Z_5/Z_{250}$ of the bioelectrical impedances, the ratio $Z_{250}/Z_5$ of the bioelectrical impedances, the ratio X/R of the reactance component X to the resistance component R, or the ratio R/X of the resistance component R to the reactance component X. The present invention is not limited to these kinds of examples. For example, the condition score may be calculated on the basis of the ratio of resistance components. For example, the average value $\mu x$ and the standard deviation value $\sigma x$ may be calculated by using the ratio $R_5/R_{250}$ or $R_{250}/R_5$ of resistance components, where $R_5$ is a resistance component at the time of impressing the alternating current of the frequency of 5 kHz, and $R_{250}$ is a resistance component at the time of impressing the alternating current of the frequency of 250 kHz. After calculating the average value $\mu x$ and the standard deviation value $\sigma x$, the condition score Ti may be calculated on the basis of the average value $\mu x$ and the standard deviation value $\sigma x$ in the same way as each embodiment and each variation.

VARIATION 10

In each embodiment and each variation, information on whether or not injury or disease occurs, physical condition, or feeling is input as the additional information. Body information at the time of calculating the bioelectrical impedance such as the user's weight, body temperature, blood pressure and the like may be recorded as the additional information. For example, since the user's weight is calculated when the user steps on the main unit 2, the calculated weight may be recorded as the additional information. If the weight changes +/- 2 kg within a week, a message indicating that weight rapidly changed and asking whether or not there is any problem in body conditions may be displayed on the display section whether or not there is a change in the condition score.

For example, if there is no continuous change in the condition score, and only the weight decreased, the message "Score does not change, but weight decreased. Is there any change in body condition?" may be displayed. If the condition score changes continuously, and the weight decreases, the message "Both condition score and weight declined. Is there any change in body condition?" may be displayed.

The user's body temperature or blood pressure may be recorded as the additional information. For example, after calculating the average of the user's body temperatures or blood pressure for a few days, if the user's body temperature or blood pressure is over the average value +/- 2SD, in which D is standard deviation, an advice message like the above-described message may be displayed.

VARIATION 11

In the first embodiment, the fifth embodiment, and the variation 1, the measured voltage and the reference current are obtained by the control section 31 as an obtaining section, so that the bioelectrical impedance is calculated on the basis of the obtained voltage and reference current by the operating section 38. The present invention is not limited to this kind of example.

For example, in the first embodiment and the fifth embodiment, the bioelectrical impedances may be measured outside the control section 31, so that the measured bioelectrical impedances may be obtained by the control section 31 as the obtaining section. Then, the ratio of the bioelectrical impedances may be calculated on the basis of the obtained bioelectrical impedances by the operating section 38.

In the first embodiment and the fifth embodiment, the ratio of bioelectrical impedances may be calculated outside the control section 31, so that the calculated ratio of bioelectrical impedances may be obtained by the control section 31 as the obtaining section. Then, the condition score may be calculated on the basis of the obtained ratio of the bioelectrical impedances and the past ratios of the bioelectrical impedances recorded as the measurement history by the operating section 38.

In the third embodiment, the fourth embodiment, and the variation 1, the bioelectrical impedances, which are sent from the body composition analyzer 200, are obtained by the control section 50 as the obtaining section. Then, the ratio of the bioelectrical impedances is calculated on the basis of the obtained bioelectrical impedances by the operating section 38. The present invention is not limited to this kind of example.

For example, in the third embodiment and the fourth embodiment, the measured voltage and the reference current may be sent from the body composition analyzer 200, so that the sent voltage and reference current may be obtained by the control section 50 as the obtaining section. Then, the bioelectrical impedances may be calculated on the basis of the obtained voltage and reference current by the operating section 38.

In the third embodiment and the fourth embodiment, the measured ratio of bioelectrical impedances may be sent from the body composition analyzer 200, so that the sent ratio of bioelectrical impedances may be obtained by the control section 50 as the obtaining section. Then, the condition score may be calculated on the basis of the obtained ratio of the bioelectrical impedances and the past ratios of the bioelectrical impedances recorded as the measurement history by the operating section 38.

VARIATION 12

In the second embodiment and the variation 2, the measured voltage and the reference current are obtained by the control section 31 as the obtaining section, so that the operating section 38 executes a waveform processing on the basis of the obtained voltage and reference current to calculate the reactance component X and the resistance component R in the bioelectrical impedance. Then, the ratio X/R or R/X of the reactance component X and the resistance component R is executed, so that the condition score is calculated on the basis of the calculated ratio X/R or R/X and the past ratios X/R or R/X recorded as the measurement history. The present invention is not limited to this kind of example.

For example, the reactance component X and the resistance component R in the bioelectrical impedance may be calculated outside the control section 31, so that the calculated reactance component X and resistance component R may be obtained by the control section 31 as the obtaining section. Then, the ratio X/R or R/X may be calculated on the basis of the obtained reactance component X and resistance component R by the operating section 38.

In the variation 3, in the case of calculating the condition score on the basis of the ratio X/R or R/X of the reactance component X and the resistance component R, the information obtained by the control section is not limited to the measured voltage and the reference current. For example, the ratio X/R or R/X of the reactance component X and the resistance component R may be calculated outside the control section, so that the calculated ratio X/R or R/X may be obtained by the control section as the obtaining section.

In the apparatus described in the fifth embodiment, in the case of calculating the condition score on the basis of the ratio X/R or R/X of the reactance component X and the resistance component R, the measured voltage and the reference current may be obtained by the control section 31 as the obtaining section. The ratio X/R or R/X may be calculated outside the control section 31, so that the ratio X/R or R/X may be obtained by the control section 31 as the obtaining section.

In the apparatus described in the third embodiment and the fourth embodiment, in the case of calculating the condition score on the basis of the ratio X/R or R/X of the reactance component X and the resistance component R, the measured voltage and the reference current may be sent from the body composition analyzer 200, so that the sent voltage and reference current may be obtained by the control section 50 as the obtaining section. The ratio X/R or R/X may be sent from the body composition analyzer 200, so that the sent ratio X/R or R/X may be obtained by the control section 50 as the obtaining section.

In the second embodiment, the ratio X/R of the reactance component X to the resistance component R in the bioelectrical impedance at the time of impressing the alternating current of the single frequency of 50 kHz, which is correlation with the ratio of the bioelectrical impedance at the time of impressing the alternating current of low frequency of 5 kHz to the ratio of the bioelectrical impedance at the time of impressing the alternating current of high frequency of 250 kHz. The present invention is not limited to this kind of example. The ratio X/R of the reactance component X to the resistance component R in the bioelectrical impedance at the time of impressing the alternating current of another single frequency, which is a correlation with the ratio of the bioelectrical impedance at the time of impressing the alternating current of low frequency of 1 to 10 kHz to the ratio of the bioelectrical impedance at the time of impressing the alternating current of high frequency of 200 to 1000 kHz.

Description of reference numerals

100A, 100B, 100C, 100D:      Condition Information Processing Apparatus

| 2: | Main Unit |
|---|---|
| 2a: | Covering Member |
| 2b: | Bottom Board Member |
| 21, 51: | Display Section |
| 22, 52: | Input Section |
| 22a: | Setting Key |
| 22b: | Up Key |
| 22c: | Down Key |
| 23, 24: | Bioelectrical Impedance Measuring Electrode |
| 23a: | First Current Applying Electrode |
| 23b: | Second Current Applying Electrode |
| 24a: | First Voltage Impressing Electrode |
| 24b: | Second Voltage Impressing Electrode |
| 31, 50: | Control Section |
| 32: | Current Generating Section |
| 32a: | Reference Current Detecting Section |
| 32b: | Alternating Current Applying Section |
| 32c: | Frequency Setting Section |
| 33: | Voltage Measuring Section |
| 34, 57: | Memory Section |
| 34a, 57a: | RAM |
| 34b, 57b: | ROM |
| 35: | A/D Converting Section |
| 36: | Weight Measuring Section |
| 37, 53: | Power Supply Section |
| 38, 58: | Operating Section |
| 38a, 58a. 68: | Condition Estimating Section |
| 200: | Body Composition Analyzer |

**Claims**

1. A condition information processing apparatus comprising:

   an obtaining section adapted for obtaining information relating to a bioelectrical impedance measured by means of impressing an alternating current of a predetermined frequency to a body surface of a user; and
   an operating section adapted for calculating an index relating to a body condition on the basis of the information relating to the bioelectrical impedance.

2. The condition information processing apparatus according to claim 1, further comprising a condition estimating section adapted for estimating the body condition on the basis of a newly calculated index relating to the body condition and a past index relating to the body condition recorded as a measurement history.

3. The condition information processing apparatus according to claim 1 or 2, further comprising:

   a pair of current applying electrodes that is capable of contacting the body surface;
   a pair of voltage measuring electrodes that is capable of contacting the body surface;
   an alternating current applying section adapted for applying an alternating current to the current applying electrodes;
   a frequency setting section adapted for setting a predetermined frequency as a frequency of the alternating current impressed by the alternating current applying section; and
   a voltage measuring section adapted for measuring a voltage at the time of impressing the alternating current of the predetermined frequency,
   wherein the obtaining section obtains either of the voltage measured by the voltage measuring section and the bioelectrical impedance as the information relating to the bioelectrical impedance.

4. The condition information processing apparatus according to any one of claims 1 to 3,
   wherein the obtaining section obtains either of: a) the voltage measured by means of impressing the alternating current of a predetermined low frequency and the alternating current of a predetermined high frequency, and b) the bioelectrical impedance as the information relating to the bioelectrical impedance, and
   the operating section calculates the index relating to the body condition on the basis of a ratio of the bioelectrical impedance at the time of impressing the alternating current of high frequency and the bioelectrical impedance at the time of impressing the alternating current of low frequency.

5. The condition information processing apparatus according to any one of claims 1 to 3, wherein the obtaining section obtains either of a) the voltage measured by means of impressing an alternating current of a predetermined single frequency, and b) a ratio of a resistance component and a reactance component in a bioelectrical impedance on the basis of the voltage, and
   the operating section adapted for calculating the index relating to the body condition on the basis of the ratio of the resistance component and the reactance component in the bioelectrical impedance.

6. The condition information processing apparatus according to any one of claims 1 to 5, wherein the operating section refers to additional information in the measurement history, in which the calculated indexes relating to the condition and the addition information associated with the index, and aggregates the indexes in the measurement history, which satisfy a predetermined condition, to calculate the index.

7. The condition information processing apparatus according to claim 6, wherein the additional information includes: a) an objective information including at least one of the user's weight, body temperature, and blood pressure at the time of calculating the index relating to the condition; and b) a subjective information input on the basis of an operation by the user,
   the operating section appropriately selects the objective information and the subjective information, so that the operating section aggregates the indexes associated with at least one of the selected objective information and the selected subjective information in the measurement history.

8. A non-transitory computer readable recording medium in which a program causing a computer to function as:

   an obtaining section adapted for obtaining information relating to a bioelectrical impedance measured by means of impressing an alternating current of a predetermined frequency to a body surface of a user; and

an operating section adapted for calculating an index relating to a body condition on the basis of the information relating to the bioelectrical impedance.

9. The non-transitory computer readable recording medium according to claim 8, the program further causing a computer to function as:

a condition estimating section adapted for estimating the body condition on the basis of a newly calculated index relating to the body condition and a past index relating to the body condition recorded as a measurement history.

10. A method for processing condition information comprising:

obtaining information relating to a bioelectrical impedance measured by means of impressing an alternating current of a predetermined frequency to a body surface of a user; and
calculating an index relating to a body condition on the basis of the information relating to the bioelectrical impedance.

11. The method for processing condition information according to claim 10, further comprising estimating the body condition on the basis of a newly calculated index relating to the body condition and a past index relating to the body condition recorded as a measurement history.

12. The method for processing condition information according to claim 10 or 11, further comprising:

contacting a pair of current applying electrodes to the body surface;
contacting a pair of voltage measuring electrodes to the body surface;
applying an alternating current to the current applying electrodes;
setting a predetermined frequency as a frequency of the impressed alternating current; and
measuring a voltage at the time of impressing the alternating current of the predetermined frequency;
obtaining either of the voltage measured by the voltage measuring section and the bioelectrical impedance as the information relating to the bioelectrical impedance.

# FIG. 1

# FIG. 2

## FIG. 3

RESISTANCE OF
EXTRACELLULAR
FLUID

X : MUSCLE TISSUE

RESISTANCE OF
INTRACELLULAR
FLUID

ELECTRICAL
CAPACITANCE
OF CELL
MEMBRANE

## FIG. 4

HIGH FREQUENCY    LOW FREQUENCY

INTRACELLULAR
FLUID

EXTRACELLULAR
FLUID

# FIG. 5

VOLUME V = CROSS SECTION A × LENGTH l

CROSS SECTION A

RESISTIVITY
$\rho\ \Omega$m

LENGTH l

FIG. 6

# FIG. 7

```
        ( POWER ON )
             │
             ▼
    ┌─────────────────┐  ─ S1
    │     INITIAL     │
    │     SETTING     │
    └─────────────────┘
             │
             ▼
    ╱───────────────────╲  ─ S2
   ╱  CONDITION ESTIMATING ╲
   ╲       MODE?          ╱
  NO ╲───────────────────╱
             │ YES
             ▼
    ┌─────────────────────┐  ─ S3
    │   MEASURUE WEIGHT   │
    └─────────────────────┘
             │
             ▼
    ┌─────────────────────┐  ─ S4
    │     CALCULATE       │
    │ LOW FREQUENCY IMPEDANCE │
    └─────────────────────┘
             │
             ▼
    ┌─────────────────────┐  ─ S5
    │ CALCULATE HIGH FREQUENCY │
    │      IMPEDANCE      │
    └─────────────────────┘
             │
             ▼
    ┌─────────────────────┐  ─ S6
    │ CALCULATE RATIO $Z_5 / Z_{250}$ OF │
    │ BIOELECTRICAL IMPEDANCE │
    └─────────────────────┘
             │
             ▼
    ┌─────────────────────┐  ─ S7
    │ CALCULATE CONDITION SCORE │
    └─────────────────────┘
             │
             ▼
    ┌─────────────────────┐  ─ S8
    │  ESTIMATE CONDITION │
    └─────────────────────┘
             │
             ▼
    ┌─────────────────────┐  ─ S9
    │    DISPLAY RESULT   │
    └─────────────────────┘
             │
             ▼
    ┌─────────────────────┐  ─ S10
    │ RECORD MEASUREMENT  │
    │      HISTORY        │
    └─────────────────────┘
             │
             ▼
    ╱───────────────────────╲  ─ S11
   ╱   CONDITION ESTIMATING   ╲
YES ─ MODE ON IN A PREDTERMINED │
   ╲      TIME PERIOD?        ╱
    ╲───────────────────────╱
             │ NO
             ▼
       ( POWER OFF )
```

# FIG. 8

EP 2 609 856 A2

# FIG. 9

EP 2 609 856 A2

FIG. 10

# FIG. 11

# FIG. 12

FIG. 13

EP 2 609 856 A2

FIG. 14

# FIG. 15

FIG. 16

# FIG. 17

R²=0.9465

RIGHT LEG 50kHz X/R

RIGHT LEG Z 5/250kHz

# FIG. 18

```
        ( POWER ON )
             │
             ▼
    ┌─────────────────┐
    │     INITIAL      │ ──── S1
    │     SETTING      │
    └─────────────────┘
             │
             ▼
    ╱─────────────────────╲
   ╱  CONDITION ESTIMATING ╲ ──── S2
   ╲       MODE?           ╱
  NO ╲─────────────────────╱
             │ YES
             ▼
    ┌─────────────────────┐
    │   MEASURUE WEIGHT   │ ──── S3
    └─────────────────────┘
             │
             ▼
    ┌─────────────────────┐
    │  OBTAIN VOLTAGE UPON │ ──── S20
    │ IMPRESSING ALTERNATING│
    │  CURRENT OF SINGLE   │
    │     FREQUENCY        │
    └─────────────────────┘
             │
             ▼
    ┌─────────────────────┐
    │ CALCULATE RATIO X/R OF│ ──── S21
    │ RESISTANCE COMPONENT AND│
    │  REACTANCE COMPONENT │
    └─────────────────────┘
             │
             ▼
    ┌─────────────────────┐
    │CALCULATE CONDITION SCORE│ ──── S7
    └─────────────────────┘
             │
             ▼
    ┌─────────────────────┐
    │  ESTIMATE CONDITION  │ ──── S8
    └─────────────────────┘
             │
             ▼
    ┌─────────────────────┐
    │   DISPLAY RESULT     │ ──── S9
    └─────────────────────┘
             │
             ▼
    ┌─────────────────────┐
    │ RECORD MEASUREMENT   │ ──── S10
    │     HISTORY          │
    └─────────────────────┘
             │
             ▼
    ╱─────────────────────╲
   ╱  CONDITION ESTIMATING  ╲ ──── S11
 YES ╱ MODE ON IN A PREDTERMINED╲
   ╲    TIME PERIOD?       ╱
    ╲─────────────────────╱
             │ NO
             ▼
        ( POWER OFF )
```

# FIG. 19

200

BODY
COMPOSITION
ANALYZER

100B

CONDITION
INFORMATION
PROCESSING
APPARATUS

NET

# FIG. 20

200

32

23b

23a

24a

24b

## CURRENT GENERATING SECTION

32a — REFERENCE CURRENT DETECTING SECTION

32b — ALTERNATING CURRENT APPLYING SECTION

32c — FREQUENCY SETTING SECTION

VOLTAGE MEASURING SECTION ~33

A/D CONVERTING SECTION — 35

WEIGHT MEASURING SECTION — 36

22 — INPUT SECTION

POWER SUPPLY SECTION

37

CONTROL SECTION

31

COMMUNICATION I/F — 42

OPERATING SECTION

38b

DISPLAY SECTION

21

MEMORY SECTION

RAM — 34a

34b — ROM — 34

# FIG. 21

100B

51

DISPLAY
SECTION

52

INPUT
SECTION

50

CONTROL SECTION
(INCLUDING OBTAINING SECTION)

55

COMMUNICATION
I/F

POWER
SUPPLY
SECTION

53

58

OPERATING
SECTION

58a

CONDITION
ESTIMATING
SECTION

57

MEMORY
SECTION

RAM — 57a

ROM — 57b

HDD — 57c

# FIG. 22

POWER ON

INITIAL SETTING — S30

CONDITION ESTIMATING MODE? — S31

NO

YES

CONFIRM CONNECTION WITH CONDITION INFORMATION PROCESSING APPARATUS — S32

INPUT ID — S33

MEASURE WEIGHT — S34

CALCULATE LOW FREQUENCY IMPEDANCE — S35

CALCULATE HIGH FREQUENCY IMPEDANCE — S36

DISPLAY WEIGHT — S37

SEND DATA — S38

POWER OFF

# FIG. 23

```
                    ┌─────────────┐
                    │    START    │
                    └─────────────┘
                           │
                           ▼
          ┌──────────────────────────────────┐     S40
          │     CONFIRM CONNECTION WITH       │
          │   BODY COMPOSITION ANALYZER       │
          └──────────────────────────────────┘
                           │
                           ▼
          ┌──────────────────────────────────┐     S41
          │          RECEIVE DATA             │
          └──────────────────────────────────┘
                           │
                           ▼
          ┌──────────────────────────────────┐     S42
          │   CALCULATE CONDITION SCORE       │
          └──────────────────────────────────┘
                           │
                           ▼
          ┌──────────────────────────────────┐     S43
          │        ESTIMATE CONDITION         │
          └──────────────────────────────────┘
                           │
                           ▼
          ┌──────────────────────────────────┐     S44
          │          DISPLAY RESULT           │
          └──────────────────────────────────┘
                           │
                           ▼
          ┌──────────────────────────────────┐     S45
          │    RECORD CALCULATED DATA         │
          │    ASSOCIATE WITH ID AS           │
          │    MEASUREMENT HISTORY            │
          └──────────────────────────────────┘
                           │
                           ▼
                    ┌─────────────┐
                    │     END     │
                    └─────────────┘
```

# FIG. 24

P51    P50

90
80
70
   ◇67
      ◇66
   □64
         ◇62
60     □59
        □55
50
40
30
20
10

2011/02/24  2011/03/04  2011/03/09

┌─────────────────┐
│ ─□─ RIGHT LEG   │
│ ─◇─ LEFT LEG    │
└─────────────────┘

Score is declining continuously. Are you so tired or in poor
condition? How about checking your body or resting?

P52

# FIG. 25

# FIG. 26

Score recovers to above the last score. Keep measuring body composition to observe progress.

# FIG. 27

Score is continuously rising. Keep measuring body composition to observe progress.

# FIG. 28

P51

P50

Score is over 50 points. In order to increase the score even more, keep taking care of your body and measuring body composition to observe progress.

P52

# FIG. 29

200

BODY
COMPOSITION
ANALYZER

100C

CONDITION
INFORMATION
PROCESSING
APPARATUS

NET

300

TERMINAL DEVICE

# FIG. 30

100C

51
DISPLAY
SECTION

52
INPUT
SECTION

53
POWER
SUPPLY
SECTION

50
CONTROL SECTION
(INCLUDING OBTAINING SECTION)

55
COMMUNICATION
I/F

58
OPERATING
SECTION

57
MEMORY
SECTION
57a RAM
57b ROM
57c HDD

# FIG. 31

300

61
DISPLAY
SECTION

62
INPUT
SECTION

63
POWER
SUPPLY
SECTION

60
CONTROL SECTION

65
COMMUNICATION
I/F

68
CONDITION
ESTIMATING
SECTION

67
MEMORY
SECTION
67a RAM
67b ROM
67c HDD

# FIG. 32

```
          START              S50
            │
            ▼
CONFIRM CONNECTION WITH
BODY COMPOSITION ANALYZER
            │
            ▼                 S51
      RECEIVE DATA
            │
            ▼                 S52
CALCULATE CONDITION SCORE
            │
            ▼                 S53
 RECORD CALCULATED DATA
   ASSOCIATE WITH ID AS
  MEASUREMENT HISTORY
            │
            ▼
          END
```

# FIG. 33

```
          START              S54
            │
            ▼
CONFIRM CONNECTION WITH
    TERMINAL DEVICE
            │
            ▼                 S55
RECEIVE ID AND REQUEST FOR
      SENDING DATA
            │
            ▼                 S56
       SEND DATA
            │
            ▼
          END
```

# FIG. 34

```
            ( START )
                │
                ▼
┌────────────────────────────┐   S60
│ CONFIRM CONNECTION WITH    │
│ CONDITION INFORMATION      │
│ PROCESSING APPARATUS       │
└────────────────────────────┘
                │
                ▼
┌────────────────────────────┐   S61
│         SEND ID            │
└────────────────────────────┘
                │
                ▼
┌────────────────────────────┐   S62
│  REQUEST FOR SENDING DATA  │
└────────────────────────────┘
                │
                ▼
┌────────────────────────────┐   S63
│       RECEIVE DATA         │
└────────────────────────────┘
                │
                ▼
┌────────────────────────────┐   S64
│    ESTIMATE CONDITION      │
└────────────────────────────┘
                │
                ▼
┌────────────────────────────┐   S65
│      DISPLAY RESULT        │
└────────────────────────────┘
                │
                ▼
            (  END  )
```

# FIG. 35

100D

CONDITION
INFORMATION
PROCESSING
APPARATUS

300

TERMINAL
DEVICE

NET

FIG. 36

100D

23b

23a

24a

24b

32

CURRENT GENERATING
SECTION

32a — REFERENCE CURRENT
DETECTING SECTION

32b — ALTERNATING CURRENT
APPLYING SECTION

32c — FREQUENCY SETTING
SECTION

VOLTAGE
MEASURING
SECTION — 33

A/D
CONVERTING
SECTION — 35

WEIGHT
MEASURING
SECTION — 36

22 — INPUT
SECTION

POWER
SUPPLY
SECTION

37

DISPLAY
SECTION

21

31

CONTROL SECTION
(INCLUDING OBTAINING SECTION)

MEMORY
SECTION

RAM — 34a

ROM — 34

34b

OPERATING
SECTION — 38

COMMUNICATION
I/F — 42

65

# FIG. 37

POWER ON

INITIAL SETTING — S70

CONDITION ESTIMATING MODE? — S71

NO

YES

CONFIRM CONNECTION WITH TERMINAL DEVICE — S72

INPUT ID — S73

MEASURE WEIGHT — S74

CALCULATE LOW FREQUENCY IMPEDANCE — S75

CALCULATE HIGH FREQUENCY IMPEDANCE — S76

CALCULATE RATIO $Z_5/Z_{250}$ OF BIOELECTRICAL IMPEDANCE — S77

CALCULATE CONDITION SCORE — S78

DISPLAY WEIGHT — S79

SEND DATA — S80

POWER OFF

FIG. 38

## FIG. 39

EP 2 609 856 A2

# FIG. 40

$R^2 = 0.8652$

RIGHT LEG Z 250/5kHz

RIGHT LEG 50kHz R/X

EP 2 609 856 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005077148 A